# EUROPEAN PATENT APPLICATION

(11) **EP 2 116 263 A1**
(43) Date of publication of application: **11.11.2009**
(21) Application number: 08008454.4
(22) Date of filing: 05.05.2008
(51) Int. Cl.: A61K 41/00, A61K 47/48, A61P 31/10, A61P 37/06, A61P 29/00, A61P 17/06, A61P 27/02, A61P 35/00

(54) **Reversibly light-switchable drug-conjugates**

(71) Applicant: Max-Planck-Gesellschaft zur Förderung der Wissenschaften e.V., 80539 München (DE)
(72) Inventor: Erdmann, Frank Dr., 06114 Halle (DE); Zhang, Yixin Dr., 8046 Zürich (CH); Fischer, Gunter Dr., 06120 Halle (DE)
(74) Representative: Stolmár, Matthias

(57) **Abstract**

The present invention refers to a drug conjugate of the general formula A-B-C, wherein A is a drug component, B is a photoisomerizable group and C is a small molecule protein binder. The conjugates according to the invention can be used as a medicament, especially for the treatment of inflammations, fungal, viral bacterial or other infections, cancer, vascular diseases, cardiovascular diseases, obesity, neurological disorders, degenerative neurological disorders, psychiatric diseases or conditions, depression, hormonal disorders. Furthermore, the present invention refers to a method for reversibly modulating the activity of a drug compound in cells.

## Description

The present invention refers to a drug conjugate of the general formula A-B-C, wherein A is a drug component, B is a photoisomerizable group and C is a small molecule protein binder. The conjugates according to the invention can be used as medicaments, e.g. for the treatment of inflammations, fungal, viral, bacterial or other infections. Furthermore, the present invention refers to a method for increasing the activity of a drug compound in cells.

The medicinal efficacy and side effects of a drug molecule are not only determined by its activity and specificity to its physiological and/or pathological targets, but also by the bio-distributions and local activity of the drugs within the human body. In an ideal case, an active drug would be localized only at the site of disease and exert its therapeutic effects locally. The high ratio of drug activity in disease tissue to healthy tissue would lead to maximized therapeutic efficacy, as well as minimized side effect on other parts of the body.

In the field of pharmacology and drug design, a great number of novel approaches are emerging which allow to design, to synthesize, and to screen molecules with high activity and specificity to certain targets of physiological and/or pathological interest. However, methods for tuning and improving drug local activity still represent a great challenge.

There are a number of approaches to improve the local concentration and activity of a certain drug. For example, intimate contact with the blood makes the tumor endothelial cells a uniquely accessible target within the tumor (Neri and Bicknell, 2005). Furthermore, given that both inflammation and growth of solid tumors are often dependent on their capacity to acquire a blood supply, much effort has been directed towards the development of agents that facilitate the detection or disruption of this process, based on unique endothelial markers associated with angiogenesis. Antibodies with high affinity to such markers have been conjugated to anti-cancer drugs, in order to achieve high local concentration of the drugs around the tumors.

Another approach to achieve high local drug activity is to generate prodrugs, which can be hydrolyzed and activated in vivo, dependent on the local conditions such as pH and enzymatic activity (Olbe et al., 2003). For example, it has been shown that extracellular pH was lower in tumors than in healthy tissue. Meanwhile, inflammatory tissue or tumor can also possess high proteolytic activity due to the over-expression of some proteases such as different matrix metalloproteinases.

While vascular targeting and prodrugs are both valuable approaches to improve the pharmaceutical profile of a certain drug, they have their disadvantages. The high production costs as well as the potential risk of immunogenicity are great challenges for protein-based therapeutic agents. Moreover, the amount of drug molecules that can be delivered by antibody is limited by the amount of antigen presented around the disease tissue. Although an advantage of antibody-based agents is that the binding of antibody to the vascular antigen is reversible and the tissue specificity is retained as the antibody conjugate re-enters the disease site, many drugs need to enter into cells for exerting their effects.

For example, for the immunophilin-dependent immunosuppressive drugs (Fischer, 1994) such as cyclosporin A, FK506, and rapamycin, both their presenter proteins and immunological targets are intracellular proteins. Since it is very difficult to deliver therapeutic proteins into cells, an antibody-drug conjugate should always be designed as a prodrug, with a cleavable linker that enables the release of cell permeable active compound. In the case of prodrugs, both pH condition and proteolytic activity do not represent exclusively unique properties associated with a disease tissue. For example, a low pH environment can be found in organs such as kidney, while some healthy tissues also exhibit high proteolytic activity.

In recent years, a photo-activation strategy has been proposed (Bednarski et al., 2007), in which the prodrug contains a photo-labile chemical bond. Thus, the deprotection of prodrugs and formation of active compounds is directed by light irradiation. Nevertheless, given that the transformation from an inactive prodrug to an active drug is not reversible, the active compound will be circulated through blood stream and brought to other healthy sites. This results in unwanted side-effects of the treatment.

A drug used for the treatment of various diseases is cyclosporin A. Cyclosporin A (CsA) is a cyclic undecapeptide (figure **1**) that was first isolated from soil samples in the 1970s and produced by *Tolypocladium inflatum* (Borel, 1990). It exhibits many biological activities including antiinflammatory, anti-fungal, anti-parasitic as well as immunosuppressive activities. It is commonly used in transplantation surgery and in the treatment of autoimmune diseases (Sandimmune®). The clinical applications of CsA have shown that treatment with CsA is accompanied by significant side effects such as hypertension and nephrotoxicity (Busauschina et al., 2004). Therefore, it remains a great challenge to increase the tissue/organ specificity, in order to diminish the un-favorite side-effects.

Furthermore, CsA remains one of the most effective and rapidly acting treatments currently available for psoriasis (Berth-Jones, 2005; Ho, 2004). An animal model of psoriasis in mice has been established by Parker and the coworkers (Schon et al., 1997). Virtually all the diverse manifestations of this disease can respond. The main side effects are nephrotoxicity and hypertension. CsA should be used in single or intermittent short courses for all except the most severe cases as this is safer than continuous treatment. The rate of improvement depends very much on the dose, which ranges from 2 to 5.0 mg/kg/day. CsA can be combined with any topical treatment and a useful dose-sparing effect can be achieved in this way if patients are compliant. In severe cases cyclosporin is often used in combination with other systemic antipsoriatic drugs in order to spare the dose of each agent and reduce toxicity. Skin represents the tissue most suitable for light associated treatment. However, the concurrent or intercurrent use of ultraviolet therapy is discouraged due to the increased risk of non-melanoma skin cancer.

Furthermore, CsA can influence the intracellular signaling and the activities of several transcription factors occurring in inflamed tissues. One of those transcription factors is the nuclear factor of activated T cells (NFAT). Different isoforms of NFAT play diverse roles in a variety of processes in the immune system and other tissues (Graef et al., 2001).

Pharmacologic interference of CsA or FK506 (Tacrolimus®) with the calcineurin/NFAT pathway is also effective in various autoimmune disorders, including arthritis (Kitahara and Kawai, 2007) and air way inflammation. The most convincing evidence that NFATs may be important in the pathogenesis or perpetuation of inflammatory arthropathies stems from the observation that treatment with CsA is effective in otherwise refractory rheumatoid arthritis. Recently, direct experimental evidence has also implicated specific NFAT members including: (1) NFAT5 was found to be expressed specifically in the RA synovial lining, (2) in the serum transfer model of arthritis, which typically features a symmetric polyarthritis, the NFAT1 KO and the NFAT1/4 double KO mice developed an asymmetric oligoarthritis, and (3) immunohistologic studies have revealed wide-spread expression of NFAT1-4 in the synovia from patients with inflammatory arthropathies.

Moreover, many recent evidences have suggested that inflammation is the key factor in the pathogenesis of various ocular surface diseases which interact with various genetic, environmental, and psychosocial factors (Scheinfeld, 2007). Topical corticosteroids, in spite of their associated side effects, are the major treatments for patients with vision threatening disease. Recently, topical cyclosporin preparations were approved by the FDA and EMEA and became available for use in ophthalmology. Cyclosporin A 0.05% ocular emulsion (eye drops) is proved for treating dry eyes including dry eyes caused by collagen vascular disease. Cyclosporin A eye drops might also have utility in treating eye pathology of ocular rosacea, atopic keratoconjunctivitis, graft versus host disease, herpes keratitis, chronic sarcoidosis of the conjunctiva, conjunctival manifestations of actinic prurigo, keratitis of keratitis-ichthyosis deafness (KID) syndrome, and lichen planus-related keratoconjunctivitis. Therefore, a need exists to provide cyclosporine A derivatives which exert their drug activity only in the eye.

In last years, many studies have shown that cyclosporin A can play a role in the therapy of malign and benign tumor diseases. In the treatment of solid tumors, squamous tumors and tumors of the lymphatic or hematopoietic system two strategies were pursued. The p-glycoprotein, which is in cells responsible for a rapid efflux of many drugs, is potently blocked by cyclosporin A (Foxwell et al., 1989; Ryffel et al., 1991). Inhibitors of this ABC transporter were used as adjuvants in therapy with antitumor drugs. The reduced drug-efflux leads to higher drug concentrations in cells, less side effects and higher efficacies in therapy. In many clinical trials positive effects were observed, for example in the treatment of ovarian cancer, breast cancer, and lung cancer (Knight et al., 2004; Morgan et al., 2007; Xia et al., 2007).

Another mechanism to suppress tumor growth is the inhibition of PPIases from cyclophilin type and/or the protein phosphatase calcineurin. For example cyclosporin A can suppress prostate cancer cell growth by inhibition of cyclophilin 40 (Cyp40) which interacts with and regulates presumably the androgen receptor (Periyasamy et al., 2007). In some cases, the curative effects of cyclosporin A in patients with e.g. T cell acute lymphoblastic leukemia and angioimmunoblastic T cell lymphoma can be explained by inhibition of calcineurin, in other cases, the molecular basis is still unclear as in the case of osteosarcoma and corneal squamous cell carcinoma (Advani et al., 2007; Medyouf et al., 2007; Oh et al., 2006; Tunc and Erbilen, 2006).

It is therefore an objective of the present invention to provide active compounds in order to achieve the highest therapeutic efficacy in the disease tissue and to minimize the side effects in other organs. Especially, it is an objective to provide a site-specific active compounds which fulfill the following criteria: 1) the active component of the active compound is produced or recruited only within the site of disease; 2) the active compound turns inactive in other sites of the body, because circulation of a locally activated drug through blood to other healthy organs would still cause adverse effects; 3) the activation and inactivation is reversible, which means that the active compound regains its activity as reentering the disease tissue.

The above defined objectives have been solved by a drug conjugate of the general formula A-B-C, wherein A is a drug component, B is a photoisomerizable group and C is a small molecule protein binder. The conjugates according to the present invention can be used for the treatment of various diseases as defined in the claims and described further below. In addition, the conjugates according to the invention can be used for increasing the activity of a drug compound in cells as defined in the claims.

It has been unexpectedly found that a reversible photochemical isomerization reaction is suitable to switch the conjugates according to the invention from an inactive conformation to an active conformation. This can advantageously be achieved by using near UV (e.g. 370 nm) or near IR (e.g. 740 nm) irradiation. In order to diminish the side effect caused by UV irradiation, IR irradiation can advantageously used according to the present invention. IR irradiation is especially advantageously, as IR light can penetrate through skins and, joints and even the skulls of brain (Cahalan et al., 2002).

As the inactive conformation of the conjugates according to the invention is thermally stable, the activation of the conjugates according to the invention can advantageously be directed by light irradiation to the sites of disease, while the conjugates will turn inactive in other parts of the body through thermal re-equilibration, where no irradiation is applied. This allows reducing negative side effects in healthy parts of the body, which are either not transparent to light, or not irradiated. Thus, the reversible activation and inactivation of the conjugates according to the present invention is suitable to overcome the disadvantages of conventional prodrug strategy, in which the conversion of prodrug to active drug is irreversible. The conjugate according to the invention gain their activity when reentering the irradiated site of the disease. Moreover, given that modern optical technology allows very precise 3-D localization, it is very easy to direct the light and to trigger the photoisomerization process according to the invention within a sub-cellular location.

Although a conformational difference between 2 isomers often results in only minor differences of their biological activities, the present invention provides a general strategy to design compounds with significant differences in their activities with respect to the two photoisomerizable conformational states. In other words, the active compounds according to the present invention are photoswitchable or photo-regulatable.

A further advantage of the conjugates according to the invention is provided by its small molecule protein binding moiety C which allows to recruit an assistant protein and to increase the functional difference between two photo-switchable conformations. In order to maximize the effect of this recruiting approach, the drug component A and the small molecule protein binder C are placed on the two opposite sites of the photo-responsive moiety or in other terms the photo-isomerizable group B. Thus, the photoisomerization of the photo-isomerizable group B can generate a maximal difference of activity between the active and inactive conjugate regarding the topological relationship between the drug component A and the complex of the assistant protein and its small molecule protein binder C.

The present invention is not limited to any particular drugs or diseases. An especially advantageous drug, which can be photo-regulated according to the present invention, is the immunosuppressive drug cyclosporin A (CsA). Advantageously, the conjugates comprising cyclosporin A derivatives described herein can be activated by near IR light.

It is known that the CaN-NFAT (nuclear factor of activated T-cells) pathway is targeted by CsA. Calcium/calmodulin-dependent serine/threonine protein phosphatase calcineurin (CaN) plays a pivotal role in T cell signaling and is inhibited within the treatment with immunosuppressive drug cyclosporin A (CsA) and FK506 (Liu et al., 1991). The gain-of-function mechanism of CsA in immunosuppression and its structure-activity relationship have been well understood: CsA binds to and inhibits the peptidyl prolyl *cis*/*trans* isomerase (PPIase) cyclophilin 18 (Cyp18), and the resulting Cyp18/CsA complex targets CaN. Whereas immunosuppression and CaN inhibition are associated with the Cyp18/CsA/CaN tertiary complex formation, neither CsA nor Cyp18 alone causes any detectable inhibition of CaN (Fischer, 1994; Schreiber and Crabtree, 1992). After CaN activation, dephosphorylated NFATs translocate into the nucleus and bind to specific regulatory DNA sequences. The nuclear localized and activated NFAT is then responsible for the gene transcription such as IL-2 and TNF-α.

The present invention provides advantageous photoisomerizable immunosuppressive conjugates for targeting the CaN-NFAT pathway. In order to realize reversible modulation of CaN phosphatase activity in cells, the conformations and CaN inhibitory potencies of the conjugates according to the invention can be controlled by light irradiation. For example, azobenzene (AB) can be used in preferred embodiments as the photo-responsive group of a conjugate containing CsA or CsA derivatives, since photo-isomerization of AB has been realized as a simple technique to generate conformational changes within biomolecules (Renner and Moroder, 2006). Position 8 of CsA is especially advantageous for modification (compare figure 1) because changes at this residue can lead to different influences on Cyp18/CsA/CaN complex formation (Zhang et al., 2005).

Moreover, the above described CsA derivatives exhibit distinct effects on other cyclophilin-CaN systems, because the modifications on CsA residue 8 were designed to modulate the interface between human Cyp18 and human CaN (Huai et al., 2002; Jin and Harrison, 2002). Accordingly, a modification which decreases the interactions between the human-Cyp18/CsA derivative complex and human-CaN, and hence results in non-CaN inhibitory and non-immunosuppressive CsA analogues, can still provide CsA derivatives which present the gain-of-function inhibition of CaN activity in a system with other Cyp enzymes (human or from other eukaryotic origin) and/or other CaN (human or from other eukaryotic origin).

For example, CaN pathway is also intricately involved in the growth and pathogenesis of the three major fungal pathogens of humans, *Cryptococcus neoformans, Candida albicans* and *Aspergillus fumigatus* (Steinbach et al., 2007). For 1998, the projected average incidence was 306 per million US population, with candidiasis accounting for 75 % of cases (Wilson et al., 2002). The estimated total direct cost was $2.6 billion and the average per-patient attributable cost was $31,200. Given that fungi possess a cyclophilin-calcineurin systems very different from human, and CaN activity and pathology of the fungus can be inhibited by CsA with a gain-of-function mechanism, certain CsA residue 8 modified derivatives can inhibit the CaN in such lower eukaryotic system specifically.

On the one hand, the CsA analogue can use the fungus cyclophilin as presenter protein, to inhibit the fungus calcineurin, on the other hand, the physical properties of interface between a fungus cyclophilin and a fungous calcineurin must be very different from that of human. Thus, certain cyclosporin A derivatives can inhibit the fungi calcineurin without interfering with human calcineurin and causing immunosuppression.

The present invention refers in general to three-component conjugates of the general formula A-B-C, in which a drug molecule A and a small molecule protein binder are connected by a photoisomerizable group B. The two conformations (active and inactive form) of the resulting conjugate have different pharmaceutical activities. Preferably, the conjugate is turned into the more active conformation by radiation. However, it is also possible to use conjugates which are turned into the less active form by radiation.

In order to achieve a photoswitching of the conjugates according to the invention under biocompatible condition, 2-photon photoreaction with near IR light are preferred, because near UV light can cause various unfavorable photochemical reactions in cellular environments, e.g. DNA damages (Serafini and Schellhorn, 1999) and membrane lipid peroxidation.

Both, broadband light source and light emitting diode (LED) or laser can be used for irradiation in combination with the photoswitchable conjugates of the present invention in medical treatments. The broadband light sources include, but are not limited to sunlight and arc lamps. The arc lamps include, but are not limited to neon, argon, xenon, krypton, sodium, metal halide, and mercury lamps. A monochromator is used together with a broadband light source in order to obtain a light of certain wavelength. Various LED lamps include, but are not limited to devices using inorganic semiconductor material such as gallium and/or indium containing semiconductors, silicon carbide, silicon, Sapphire, zinc selenide, and diamond; and organic semiconductor material made of either small organic molecule in a crystalline phase or polymers.

Lasers as light source include, but are not limited to, semiconductor lasers, dye lasers, gas lasers, chemical lasers, excimer lasers, solid-state lasers, and free electron lasers. Gas lasers include, but not limit to, helium-neon lasers, carbon dioxide lasers, argon-ion lasers, carbon monoxide lasers, TEA (transverse electrical discharge in gas at atmospheric pressure) lasers, and metal ion lasers; chemical lasers include, but are not limited to hydrogen fluoride laser, and deuterium fluoride laser. Excimer lasers include, but are not limited to fluorine lasers, noble gas compound lasers, KrCl laser, KrF laser, XeCl laser, and XeF laser. Solid-state lasers include, but are not limitd to neodymium laser (e.g. Nd:YVO₄, Nd:YLF, and Nd:YAG), ytterbium laser, holmium laser, thulium laser, erbium laser, and titanium-doped sapphire laser. Solid-state lasers also include glass or optical fiber hosted lasers. A laser light source can be used with different non-linear devices in order to obtain light of a certain wavelength.

Preferably, light irradiation at a certain wavelength in the range of between near UV and near IR is applied to induce the photo-chemical reaction of the photoswitchable conjugates according to the invention in the disease associated tissues. Preferably, light irradiation of 740 ± 30 nm is used for tissues that are transparent to near IR light.

Also preferred in the context of the present invention is the use of irradiation at a certain wavelength in the range between 320 to 400 nm to induce the photo-chemical reaction of the photoswitchable conjugates according to the invention in the disease associated tissue.
Preferably, light irradiation at a certain wavelength in the range between 700 to 780 nm is applied to induce the photo-chemical reaction of the photoswitchable conjugates according to the invention in the disease associated tissue. Also preferred is the use of light irradiation of a certain wavelength in the range between 1 mW to 1 kW. Additionally preferred is to apply light irradiation of a certain wavelength direct to the cells or tissues or by using an optical transmission medium (e.g. glass in fiber optics or liquids). Preferably, light irradiation is performed as continuous or oscillating irradiation with different shape profiles (e.g. sawtooth, rectangle, and triangle). A further preferred possibility is to apply light irradiation to the cells or tissues in a one- or multi-dimensional direction.

Light irradiation of a certain wavelength is applied to the site of disease tissue, preferably for treating inflammations, fungal, viral, bacterial or other infections, cancer, vascular diseases, cardiovascular diseases, obesity, neurological disorders, degenerative neurological disorders, psychiatric diseases or conditions, depression, or hormonal disorders. Preferably, irradiation is applied to the skin when the disease is an inflammation or fungal infection of the skin; irradiation is applied to the eyes when the disease is a ocular disease; irradiation is applied to joints when the disease is arthritis or another related joint inflammation; in the case of cancer, irradiation is applied to sites close to the tumor, which allows most efficient light transmission.

The photoswitchable immunosuppressive conjugates according to the present invention can advantageously be used for both clinical treatment of autoimmune disease and basic research on signal transduction. In particular, the conjugates are very useful for inducing an immunosuppressive effect on tissues which are accessible or transparent to light irradiation, for example, the skin, eyes, joints, and body cavities.

In addition, because joint tissue has a very good transparency to near IR light, the immunosuppressive conjugates according to the invention can advantageously be used for the treatment of arthritis, wherein the activity can be switch by near IR light. When only the joints are irradiated, only the compounds which circulate to the joints will be activated by the near IR light, while the compounds at other sites of the body remain inactive. Moreover, when the activated compound circulate to other tissues, where no light irradiation is applied, the conjugates will switch back to the inactive form, thus, the side-effects caused by CaN inhibition can be minimized. Moreover, the conjugates can become active again when they enter into the joints where light irradiation is present.

Another application for the conjugates according to the present invention is the treatment of eye disease, because the eye is easily accessible by light and near IR light is not visible for the human eyes. Photo-activation of immunosuppressive conjugates in the eyes using near IR light irradiation, therefore, provides an advantageous approach for achieving high therapeutic efficacy in inflammatory tissue with minimal side effects in other parts of the body.

A further application for the new conjugates is the treatment of cancer. In this case, the conjugates according to the invention can be applied topically or systemically. A light irradiation can be performed by direct or minimal invasive techniques. This procedure allows the broad or punctual irradiation of the most tumor diseases. Thus, the inhibitory and therapeutic effect of the conjugate will be activated only at the irradiated site of tumor.

Yet another application for the conjugates according to the invention is their use as an advanced tool in studying the dynamics of signal transduction. The dynamics of cytosolic calcium plays an essential role in T cell signaling (Gwack et al., 2007). Calcium clamp technique based on irreversible activation of calcium channel, electrophysiological method as patch clamp, as well as the use of calcium fluorescence indicators have been developed as advanced tools for controlling and monitoring calcium concentration in cells (Dolmetsch et al., 1998). Given that calcium is a universal signaling molecule and triggers a number of diverse pathways, a reversible regulation of single downstream target selectively would allow to studying a subset of the dynamic events. Calcium/calmodulin-dependent serine/threonine protein phosphatase calcineurin (CaN) plays a pivotal role in T cell signaling and is inhibited in the treatments of immunosuppressive drug cyclosporin A (CsA) and FK506. While the function of the dynamics of calcium signal has been studied intensively, our method provides a deep insight into a subset of the signaling cascades initiated by the calcium dynamics, namely the CaN-NFAT pathway that can be inhibited by CsA and its derivatives.

The drug component A may be any drug compound that can be chemically bonded to the photoisomerizable group B and where the activity of the drug component A depends on the conformation of the conjugate.

Although the drug component A, e.g. CsA, is conjugated to a photo-responsive moiety, specifically to a photoisomerizable moiety B (group), it is the core and the pharmacologic active component of the conjugate. For example, if CsA is used as the drug component, the conjugate forms a complex with cytosolic protein peptidyl-prolyl *cis-trans* isomerase cyclophilin and inhibits Ser/Thr phosphatase calcineurin by the binary complex. The inhibition of calcineurin results in immunosuppression and other biological activity of CsA.

Preferably, the drug A is conjugated to the photoisomerizable moiety B at the position which provides the highest activity. The most suitable position for coupling the drug A to the photoisomerizable group B can be chosen by a skilled person, based on its knowledge about the structure-activity relationship of the drug molecule. On the one hand, to prevent a reduction of the drug activity, the drug component A should not be coupled to the photoisomerizable group B at its active site; on the other hand, the photoisomerizable moiety B shall be introduced to a site close to the drug active site, thus, the two photo-switchable structures (the so-called "double") of the photoisomerizable moiety can exhibit a significant influence on the drug activity. In the case of CsA, the position 8 has been chosen for modification because changes at this residue have proven to exhibit subtle effects on Cyp18/CsA/CaN complex formations, while most changes on other positions of CsA abolish its immunosuppressive activity (Zhang et al., 2005).

Preferably, the drug component A is selected from the group consisting of cyclosporin A derivatives, FK506 (tacrolimus), rapamycin (sirolimus), pimecrolimus, everolimus, sanglifehrin, and derivatives thereof. It is further preferred that the drug component A is cyclosporin A or a cyclosporin A derivative.

Preferably, the cyclosporin A derivative is represented by the general formula **1**: 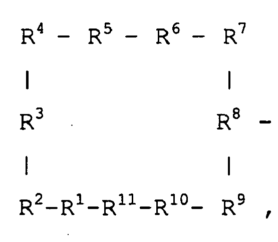 wherein
R¹ is N-methyl (4R)-4-[(E)-2-butenyl]-4-methyl-L-threonin (MeBMT) or dihydro-MeBMT;
R² is alpha-aminobutyric acid (Abu) or a halogenated derivative thereof;
R³ is N-methyl glycine (Sar), or N-methyl-D-alanine, or a halogenated derivative thereof;
R⁴, R⁶, R⁹, and R¹⁰ are N-methyl-L-leucin (MeLeu), or a halogenated derivative thereof;
R⁵ is L-valine (Val) or a halogenated derivative thereof;
R⁷ is L-alanine (Ala) or a halogenated derivative thereof;
R¹¹ is N-methyl valine (MeVal) or a halogenated derivative thereof; and wherein
R⁸ is a moiety represented by formula **2**: 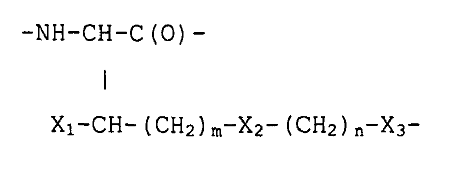 or 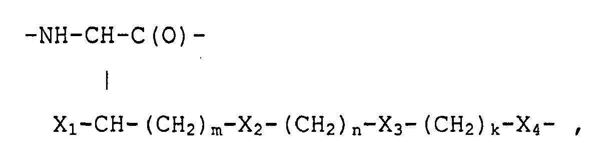 wherein
X₁ is H, methyl, and ethyl;
m is a value between 0 and 3;
X₂ is O or S;
n is a value between 0 and 4;
X₃ and X₄ is -NR₁₂-, -NR₁₂C(O)-, -NR₁₂C(S)-, -C(O)NR₁₂-, or -
C(S)NR₁₂-;
R₁₂ is H, methyl, ethyl, or propyl; and
k is a value between 0 and 7.

It is even further preferred that the residues R¹-R⁷ and R⁹-R¹¹ in formula 1 are defined as follows:
R¹ is methyl (4R)-4-[(E)-2-butenyl]-4-methyl-L-threonin (MeBMT);
R² is alpha-aminobutyric acid (Abu);
R³ is N-methyl glycine (Sar);
R⁴, R⁶, R⁹, and R¹⁰ are MeLeu;
R⁵ is Val;
R⁷ is Ala; and
R¹¹ is MeVal.

The cyclosporin A derivative of a conjugate according to the invention is represented in preferred embodiments by formula 12: 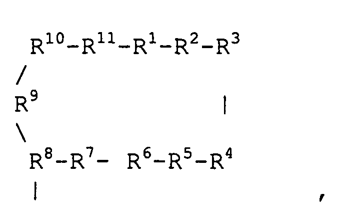 wherein R¹ is N-Methyl-(4R)-4-[(E)-2-butenyl]-4-methyl-L-threonin (MeBMT) or dihydro-MeBMT;
R² is α-aminobutyric acid (Abu) or a fluorinated derivative thereof which is fluorinated on one or position of the side chain;
R³ is N-methyl-D-alanine (D-MeAla) or N-methyl-glycine (sarcosine (Sar)) or a fluorinated derivative thereof;
R⁴, R⁶, R⁹ and R¹⁰ are independently from one another N-methyl-L-leucine (MeLeu) or a fluorinated derivative thereof;
R⁵ is L-valine (Val) or a fluorinated derivative thereof;
R⁷ is L-alanine (Ala) or a fluorinated derivative thereof;
R¹¹ is N-methyl-L-valin (MeVal) or a fluorinated derivative thereof; and
R⁸ is a moiety represented by formulae **13a and 13b:** 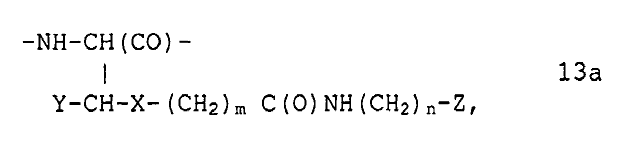 wherein X is O or S, Y is H or methyl, m has a value between 0 and 5,
n is an integer between 3 and 10,
Z is
N-R¹²R¹³ or NHC(O)OR¹⁴, wherein
R¹² is H, methyl, ethyl, n-propyl, iso-propyl or a photolysable group; and
R¹³ is H or C₁-C₈-alkyl residue, and
R¹⁴ is an acid/base labile or enzymatically cleavable group;
   or 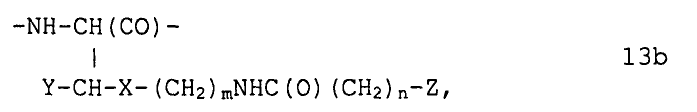 wherein X is O, S or CH₂,
Y is H or methyl,
m has a value between 0 and 5,
n is an integer between 3 and 10,
Z is N-R¹²R¹³ or NHC(O)OR¹⁴, wherein
R¹² and R¹³ are independently from one another OH, a branched, un-branched or cyclic C₁ - C₁₀ hydrocarbyl group, preferably methyl, ethyl, n-propyl, iso-propyl, a substituted aromatic group or a photolysable group and
R¹⁴ is an acid/base labile or enzymatically cleavable group thereof.

The photolysable (or in other words: photocleavable) group is an optionally further substituted nitro-aryl group, principally known from light induced DNA synthesis,

Preferably, the nitro aryl group is 2-nitroveratryloxycarbonyl, α-carboxy-2-nitrobenzyl, 1-(2-nitrophenyl)ethyl, 1-(4,5-dimethoxy-2-nitrophenyl)ethyl or 5-carboxymethoxy-2-nitrobenzyl. In a still more preferred embodiment, R²² is H.

The acid/base labile or enzymatically cleavable group is preferably a methyl, ethyl, methoxymethyl, CH₂CH₂F-, methylthiomethyl, β-glucuronide, β-galacturonide, D-glucopyranosyl, β-D-galactopyranosyl, tetra-O-acetyl-D-glucopyranosyl, or tetra-O-acetyl-β-D-galactopyranosyl group.

In a still more preferred embodiment, R² is Abu, R³ is Sar or D-Me-Ala and R⁷ is Ala. Still more preferred is an embodiment, wherein R¹ is MeBMT, R³ is Sar, R⁹, R⁶, R⁹ and R¹⁰ are MeLeu, R⁵ is L-Val and R¹¹ L-MeVal.

The photoisomerizable (or photoswitchable) group B can be any group that can undergo a reversible conformational change under light irradiation or heating and can be bound to the drug component A and the small molecule protein binder C.

Preferably, the photoisomerizable group B is selected from the group of azobenzene, stilbene, spiropyran, diarylethene, or fulgide, further preferred azobenzene.

It is additionally preferred that the azobenzene is a moiety represented by formula **3**: 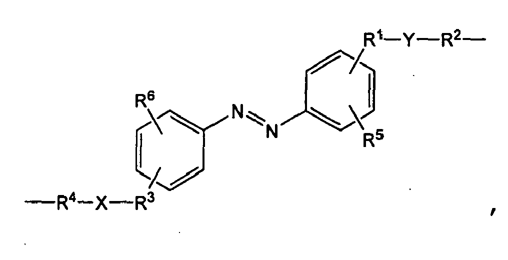 wherein
R¹, R², R³, R⁴ are independently selected from branched, un-branched or cyclic C₁ - C₁₀ hydrocarbyl groups;
X, Y are independently selected from O, S, -NR⁷-, -C(O)NR⁷-, - C(S)NR⁷-, -NR⁷(C(O)-, and -NR⁷C(S)-;
R₇ is H, methyl, ethyl, or propyl;
R⁵ and R⁶ are independently selected from H, F, Cl, Br, I, branched, un-branched or cyclic C₁ - C₁₀ hydrocarbyl groups, NO₂, CF₃, OR⁹, OCOR⁹, COOR⁹, CH₂OR⁹, CHO, COR⁹, NR⁹R¹⁰ and SR⁹;
R⁹ is H and branched, un-branched or cyclic C₁ - C₁₀ hydrocarbyl groups.

The photoswitchable N=N "double bond" can be present in its *cis* or *trans* conformation.

It is additionally preferred that the stilbene is a moiety represented by formula **4**: 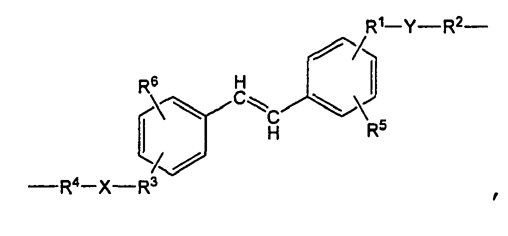 wherein
R¹, R², R³, R⁴ are independently selected from branched, un-branched or cyclic C₁ - C₁₀ hydrocarbyl groups;
X, Y are independently selected from O, S, -NR⁷-, -C(O)NR⁷-, - C(S)NR⁷-, -NR⁷(C(O)-, and -NR⁷C(S)-;
R⁵ and R⁶ are independently selected from H, F, Cl, Br, I, branched, un-branched or cyclic C₁ - C₁₀ hydrocarbyl groups, NO₂, CF₃, OR⁹, OCOR⁹, COOR⁹, CH₂OR⁹, CHO, COR⁹, NR⁹R¹⁰ and SR⁹;
R⁹ is H and branched, un-branched or cyclic C₁ - C₁₀ hydrocarbyl groups;

The photoswitchable C=C "double bond" can be present in its *cis* or *trans* conformation.

It is additionally preferred that the spiropyrane is a moiety represented by formula **5**: 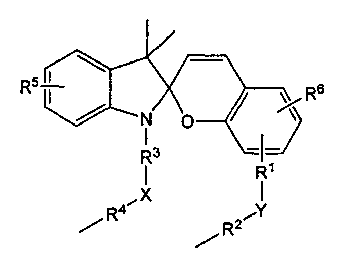 or 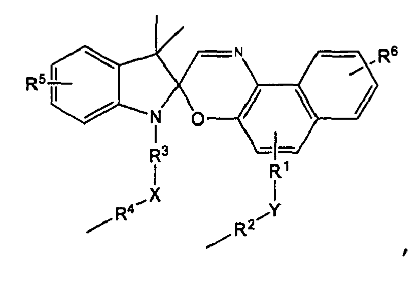 wherein
R¹, R², R³, R⁹ are independently selected from branched, un-branched or cyclic C₁-C₁₀ hydrocarbyl groups;
X, Y are independently selected from O, S, -NR⁷-, -C(O)NR⁷-, - C(S)NR⁷-, -NR⁷(C(O)-, and -NR⁷C(S)-;
R₇ is H, methyl, ethyl, or propyl;
R⁵ and R⁶ are independently selected from H, F, Cl, Br, I, branched, un-branched or cyclic C₁ - C₁₀ hydrocarbyl groups, NO₂, CF₃, OR⁹, OCOR⁹, COOR⁹, CH₂OR⁹, CHO, COR⁹, NR⁹R¹⁰ and SR⁹;
R⁹ is H and branched, un-branched or cyclic C₁ - C₁₀ hydrocarbyl groups.
   The spiropyrane may be present either in close or open configuration, which is photoswitchable.

It is additionally preferred that the diarylethene moiety is represented by the formula **6**: 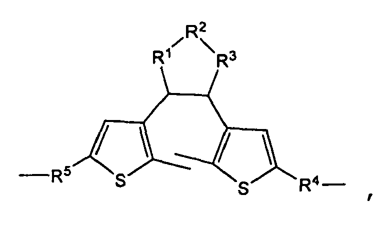 wherein
R¹, R², R³ are independently selected from CH₂, CHF, CF₂, O, S, and C(O);
R⁴ and R⁵ are independently selected from -R⁶-C(X)NR⁹-, -R⁶-R⁷-C(X)NR⁹-, -R⁶-R⁷-R⁸-C(X)NR⁹-, -R⁶-NR⁹-C(X) -, -R⁶-R⁷-NR⁹-C(X)-, - R⁶-R⁷-R⁸-NR⁹-C(X)-;
X is O or S;
R⁶, R⁷, and R⁸ are independently selected from branched, un-branched or cyclic C₁ - C₁₀ hydrocarbyl groups, -CH=C-, substitute or un-substituted phenyl or thiophene ring;
R⁹ is H, methyl, ethyl, and propyl.

The photoisomerizable moiety B can undergo a reversible structural change upon light irradiation and/or heating, while the assistant binding molecule is designed to recruit intracellular or membrane bound protein in order to amplify the structure difference between the cis/trans-isomers.

In order that the *trans* to cis photoisomerization of the conjugates according to the invention results in a significantly different pharmaceutical activity, the conjugates comprise a small molecule protein binder C which is bonded to the isomerizable group B. By introducing the small molecule protein binding moiety, an increase of the influence of a local conformational change can be provided.

The small molecule protein binder C is preferably biotin, desthiobiotin, iminobiotin; cyclosporin A and its derivative; cyclophilin binding ligands; FK506 and FKBP binding ligands; albumin binding ligands; the VIVIT peptide and derived sequences that bind to calcineurin; taxol, epothilone and their derivatives that bind to the αβ-subunit of tubulin; Strep-tag I, Strep-tag II and derived sequences that bind to streptavidin, avidin or their derivatives; a hapten or antigen that bind to monoclonal or polyclonal antibodies; poly-histidine-tag that binds to metal-containing complexes; metal/chelator-complexes that bind to poly-histidine-tag containing peptides or proteins; nucleic acids, nucleotides, and oligonucleotides that can be hybridized to other oligonucleotide sequences specifically, or can form complex with nucleotide and oligonucleotide binding proteins (such as G proteins, RNA polymerases, DNA polymerases, and transcription factors).

Further preferred, the small molecule protein binder C, is selected from biotin, desthiobiotin, and iminobiotin; cyclosporin A and its derivatives; cyclophilin binding ligands; FK506 and FKBP binding moiety; albumin binding ligands; taxol, Strep-tag I, Strep-tag II and derived sequences that bind to streptavidin or avidin; poly-histidine-tag that binds to metal-containing complexes or metal/chelator complexes that bind to poly-histidine-tag containing peptides or proteins.

Even further preferred, the small molecule protein binder C, is biotin, desthiobiotin, iminobiotin; cyclosporin A and its derivatives, or a FKBP binding moiety, further preferred a cyclosporin A derivative or a FKBP binding moiety.

It is additionally preferred that the small molecule protein binder C and its binding protein (macromolecule) show an affinity (K_{d} value) of below 200 µM. This means that it is preferred to choose the small molecule protein binder C and its binding macromolecule for a certain application so that they have a strong affinity to each other. However, if the macromolecule is highly abundant under physiological or pathological conditions, (e.g. albumin and IgG in blood), a small molecule protein binder C with moderate affinity to the binding protein is also suitable for the purposes of the present invention.

When biotin, desthiobiotin, or iminobiotin are used as the small molecule protein binder C, the conjugate is formed by amide bonds between the carboxylic group of biotin, desthiobiotin or iminobiotin and a primary or second amino group on the photoisomerizable group B.

Additionally, the cyclosporin A derivatives to be used as a small molecule protein binder are preferably represented by the general formula 1 as described above for the drug component A. Further preferred, the cyclosporin A derivatives are represented by formula **7**: 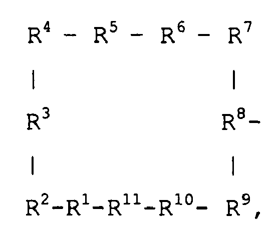 wherein
R¹ is MeBMT;
R² is Abu;
R³ is Sar;
R⁴, R⁶, R⁹, and R¹⁰ are MeLeu;
R⁵ is Val;
R⁷ is Ala;
R¹¹ is MeVal; and
R⁸ is a moiety represented by formula **8**: 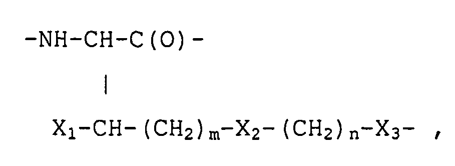 wherein
X₁ is H, methyl, and ethyl;
m is a value between 0 and 3;
X₂ is O or S;
n is a value between 0 and 4;
X₃ is -NR₁₂-, -NR₁₂C(O)-, -NR₁₂C(S)-, -C(O)NR₁₂- , or -C(S)NR₁₂-; R₁₂ is H, methyl, ethyl, or propyl.

It is additionally preferred that the FKBP binding moiety is represented by formula **9**: 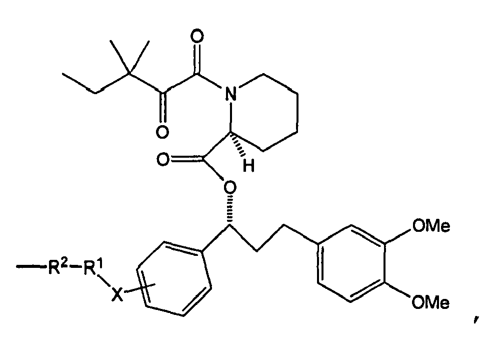 wherein
R¹ is a branched, un-branched or cyclic C₁ - C₁₀ hydrocarbyl groups;
R² is -C(X)-NR³- and -NR³-C(X)-;
R³ is H, methyl, ethyl, and propyl;
X is O or S.

According to an especially preferred embodiment of the invention, the immunosuppressive photoisomerizable conjugate A-B-C is represented by formula **10**: 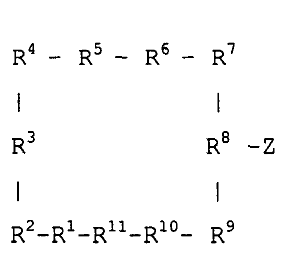
R¹ is MeBMT;
R² is Abu;
R³ is Sar;
R⁴, R⁶, R⁹, and R¹⁰ are MeLeu;
R⁵ is Val;
R⁷ is Ala;
R¹¹ is MeVal;
R⁸ is represented by formula **11**: 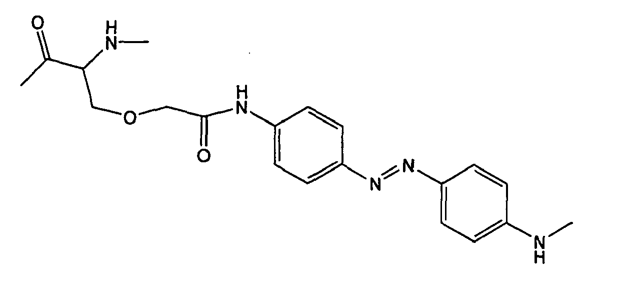 Z is a biotin, whose carboxylic acid group is coupled to the amino group of the azobenzene to form an amide bond, or a FKBP binding ligand represented by formula **9**.

For cyclosporin A derivative structures described in formula 7 and 8, when they are not conjugated to photoisomerizable group and small molecule protein binder, the conjugates are especially suited for the treatment of fungi infections, e. g. infections caused by *Cryptococcus neoformans, Candida albicans and Aspergillus fumigates.*

The conjugates according to the invention can be synthesized using standard techniques. Synthetic methodologies are also described in the examples described below.

The present invention also refers to the use of the conjugates for the manufacture of medicaments and for the treatment of diseases. The conjugates according to the invention can be used for treating inflammations, fungal, viral, bacterial or other infections, cancer, vascular diseases, cardiovascular diseases, obesity, neurological disorders, degenerative neurological disorders, psychiatric diseases or conditions, depression, hormonal disorders, preferably for treating autoimmune diseases, inflammations, and fungal, viral, bacterial or other infections. Pharmaceutical compositions comprising the conjugates according to the present invention can be prepared according to common methods known in the art. Pharmaceutical compositions and single unit dosage forms preferably comprise conjugates containing a cyclosporine A derivative or a pharmaceutically acceptable polymorph, prodrug, salt, stereoisomer, solvate, hydrate, or clathrate thereof.

Preferably, the autoimmune diseases to be treated with the conjugates according to the invention are: arthritis, psoriasis, lupus erythematosus, multiple sclerosis, autoimmune thyreopathy, autoimmune hepatitis; diabetes mellitus type I; myasthenia gravis; autoimmune polyendocrinopathy-candidiasis-ectodermal dystrophy (APECED); spondylitis ankylosans; Sjögren Syndrome; Stiff-man-Syndrome; Wegener-Klinger-Churg-Syndrome; vitiligo; autoimmune enteropathy; Goodpasture-Syndrome; and severe atopic dermatitis.

Preferably, the allergic diseases to be treated with the conjugates according to the invention are: allergic rhinitis, asthma bronchiale, allergic conjunctivitis; contact eczema; and Quincke oedema.

According to a further embodiment of the invention, the conjugates according to the invention can be used for treating malignant or benignant tumor diseases, vascular diseases, cardiovascular diseases, obesity, viral, bacterial, fungal, or other infections, neurological disorders, degenerative neurological disorders, psychiatric disease or conditions, depression, hormonal disorders, metabolic disorders, diabetes, or can be used as a drug for contraception.

Preferably, the conjugates according to the invention comprising a cyclosporine A derivative are used for treating inflammations, fungal, viral, bacterial or other infections.

According to a further embodiment of the invention, the conjugates according to the invention comprising a cyclosporine are used for treating psoriasis, arthritis, ocular diseases, HIV infections, and fungi infections.

The invention further relates to the use of the conjugates according to the invention or a pharmaceutically acceptable salt, hydrate, solvate, clathrate, prodrug or metabolite thereof, preferably in combination with an additional therapeutic agent, for treating or preventing an autoimmune disorder.

As used herein, the term "pharmaceutically acceptable salts" refers to salts prepared from pharmaceutically acceptable non-toxic acids or bases including inorganic acids and bases and organic acids and bases. Suitable pharmaceutically acceptable base addition salts for the compound of the present invention include, but are not limited to, metallic salts made from aluminium, calcium, lithium, magnesium, potassium, sodium and zinc or organic salts made from lysine, N,N'-dibenzylethylenediamine, chloroprocaine, choline, diethanolamine, ethylenediamine, meglumine (N-methylglucamine) and procaine. Suitable non-toxic acids include, but are not limited to, inorganic and organic acids such as acetic, alginic, anthranilic, benzenesulfonic, benzoic, camphorsulfonic, citric, ethenesulfonic, formic, fumaric, furoic, galacturonic, gluconic, glucuronic, glutamic, glycolic, hydrobromic, hydrochloric, isethionic, lactic, maleic, malic, mandelic, methanesulfonic, mucic, nitric, pamoic, pantothenic, phenylacetic, pivalic, phosphoric, propionic, salicylic, stearic, succinic, sulfanilic, sulfuric, tartaric acid, and p-toluenesulfonic acid. Specific non-toxic acids include hydrochloric, hydrobromic, phosphoric, sulfuric, and methanesulfonic acids. Examples of specific salts thus include hydrochloride and mesylate salts.

Individual dosage forms of the invention may be suitable for oral, mucosal (including sublingual, buccal, rectal, nasal, or vaginal), parenteral (including subcutaneous, intramuscular, bolus injection, intraarterial, intraperitoneal, intraocular, intraarticular, intracutaneous or intravenous), transdermal, or topical administration.

Examples of dosage forms include, but are not limited to: tablets; caplets; capsules, such as soft elastic gelatine capsules; cachets; troches; lozenges; dispersions; suppositories; ointments; cataplasms (poultices); pastes; powders; dressings; creams; plasters; solutions; patches; aerosols (e.g., nasal sprays or inhalers); gels; liquid dosage forms suitable for oral or mucosal administration to a patient, including suspensions (e.g., aqueous or non-aqueous liquid suspensions, oil-in-water emulsions, or a water-in-oil liquid emulsions), solutions, and elixirs; liquid dosage forms suitable for parenteral administration to a patient; and sterile solids (e. g. , crystalline or amorphous solids) that can be reconstituted to provide liquid dosage forms suitable for parenteral administration to a patient.

Parenteral dosage forms can be administered to patients by various routes including, but not limited to, subcutaneous, intravenous (including bolus injection), intramuscular, and intraarterial. Because their administration typically bypasses patients' natural defences against contaminants, parenteral dosage forms are preferably free of pyrogen and sterile or capable of being sterilized prior to administration to a patient. Examples of parenteral dosage forms include, but are not limited to, solutions ready for injection, dry products ready to be dissolved or suspended in a pharmaceutically acceptable vehicle for injection (reconstitutable powders), suspensions ready for injection, and emulsions. Suitable vehicles that can be used to provide parenteral dosage forms of the invention are well known to those skilled in the art. Examples include, but are not limited to: Water for Injection (Ph. Eur.) ; aqueous vehicles such as, but not limited to, Sodium Chloride Injection, Ringer's Injection, Dextrose Injection, Dextrose and Sodium Chloride Injection, and Lactated Ringer's Injection; water-miscible vehicles such as, but not limited to, ethyl alcohol, polyethylene glycol, and polypropylene glycol; and non-aqueous vehicles such as, but not limited to, corn oil, cottonseed oil, peanut oil, sesame oil, ethyl oleate, isopropyl myristate, and benzyl benzoate. Compounds that increase the solubility of one or more of the active ingredients disclosed herein can also be incorporated into the parenteral dosage forms of the invention.

Transdermal and topical dosage forms of the invention include, but are not limited to, creams, lotions, ointments, gels, solutions, emulsions, suspensions, or other forms known to one of skill in the art. See, e.g., "Remington's Pharmaceutical Sciences" (1990); and "Introduction to Pharmaceutical Dosage Forms", 4th ed. (Ansel, 1985). Transdermal dosage forms include "reservoir type" or "matrix type" patches, which can be applied to the skin and worn for a specific period of time to permit the penetration of a desired amount of active ingredients.

Suitable excipients (e.g., carriers and diluents) and other materials that can be used to provide transdermal and topical dosage forms encompassed by this invention are well known to those skilled in the pharmaceutical arts, and depend on the particular tissue to which a given pharmaceutical composition or dosage form will be applied. With that fact in mind, typical excipients include, but are not limited to, water, acetone, ethanol, ethylene glycol, propylene glycol, butane-1, 3-diol, isopropyl myristate, isopropyl palmitate, mineral oil, and mixtures thereof to form lotions, tinctures, creams, emulsions, gels or ointments, which are non-toxic and pharmaceutically acceptable. Moisturizers, humectants or preservatives can also be added to pharmaceutical compositions and dosage forms if desired. Examples of such additional ingredients are well known in the art. See, e.g., Remington's Pharmaceutical Sciences, 18th eds., Mack Publishing, Easton PA (1990).

Depending on the specific tissue to be treated, additional components may be used prior to, in conjunction with, or subsequent to treatment with active ingredients of the invention. For example, penetration enhancers can be used to assist in delivering the active ingredients to the tissue. Suitable penetration enhancers include, but are not limited to: acetone; various alcohols such as ethanol, oleyl, and tetrahydrofuryl; alkyl sulfoxides such as dimethyl sulfoxide; dimethyl acetamide; dimethyl formamide; polyethylene glycol; pyrrolidones such as polyvinylpyrrolidone; Kollidon grades (Povidone, Polyvidone); urea; and various water-soluble or insoluble sugar esters such as Tween 80 (polysorbate 80) and Span 60 (sorbitan monostearate).

The pH of a pharmaceutical composition or dosage form, or of the tissue to which the pharmaceutical composition or dosage form is applied, may also be adjusted to improve delivery of one or more active ingredients. Similarly, the polarity of a solvent carrier, its ionic strength, or tonicity can be adjusted to improve delivery. Compounds such as stearates can also be added to pharmaceutical compositions or dosage forms to advantageously alter the hydrophilicity or lipophilicity of one or more active ingredients so as to improve delivery. In this regard, stearates can serve as a lipid vehicle for the formulation, as an emulsifying agent or surfactant, and as a delivery-enhancing or penetration-enhancing agent. Different salts, hydrates or solvates of the active ingredients can be used to further adjust the properties of the resulting composition.

Mucosal dosage forms of the invention include, but are not limited to, ophthalmic solutions, sprays and aerosols, or other forms known to one of skill in the art. See, e.g., "Remington's Pharmaceutical Sciences", 18th eds., Mack Publishing, Easton PA (1990); and "Introduction to Pharmaceutical Dosage Forms", 4thed., Lea & Febiger, Philadelphia (1990). Dosage forms suitable for treating mucosal tissues within the oral cavity can be formulated as mouthwashes or as oral gels. In one embodiment, the aerosol comprises a carrier. In another embodiment, the aerosol is carrier free.

The conjugates according to the invention may also be administered directly to the lung by inhalation. For administration by inhalation, the compounds of this invention can be conveniently delivered to the lung by a number of different devices. For example, a Metered Dose Inhaler ("MDI") which utilizes canisters that contain a suitable low boiling propellant, e.g., dichlorodifluoromethane, trichlorofluoromethane, dichlorotetrafluoroethane, carbon dioxide or other suitable gas can be used to deliver a compound of this invention directly to the lung. MDI devices are available from a number of suppliers such as 3M Corporation, Aventis, Boehringer Ingelheim, Forest Laboratories, Glaxo-Wellcome, Schering Plough and Vectura. Alternatively, a Dry Powder Inhaler (DPI) device can be used to administer a compound of this invention to the lung. DPI devices typically use a mechanism such as a burst of gas to create a cloud of dry powder inside a container, which can then be inhaled by the patient. DPI devices are also well known in the art and can be purchased from a number of vendors which include, for example, Fisons, Glaxo-Wellcome, Inhale Therapeutic Systems, ML Laboratories, Qdose and Vectura. A popular variation is the multiple dose DPI ("MDDPI") system, which allows for the delivery of more than one therapeutic dose. MDDPI devices are available from companies such as AstraZeneca, Glaxo-Wellcome, IVAX, Schering Plough, SkyePharma and Vectura. For example, capsules and cartridges of gelatine for use in an inhaler or insufflator can be formulated containing a powder mix of the compound and a suitable powder base such as lactose or starch for these systems.

Another type of device that can be used to deliver the conjugates of this invention to the lung is a liquid spray device supplied, for example, by Aradigm Corporation. Liquid spray systems use extremely small nozzle holes to aerosolize liquid drug formulations that can then be directly inhaled into the lung. In a preferred embodiment, a nebulizer device is used to deliver the conjugates of this invention to the lung. Nebulizers create aerosols from liquid drug formulations by using, for example, ultrasonic energy to form fine particles that can be readily inhaled.

Liquid drug formulations suitable for use with nebulizers and liquid spray devices and EHD aerosol devices will typically include a conjugate of this invention with a pharmaceutically acceptable carrier. Preferably, the pharmaceutically acceptable carrier is a liquid such as alcohol, water, polyethylene glycol or a perfluorocarbon. Optionally, another material may be added to alter the aerosol properties of the solution or suspension of a cyclosporin derivative of this invention. Preferably, this material is liquid such as an alcohol, glycol, polyglycol or a fatty acid. Other methods of formulating liquid drug solutions or suspension suitable for use in aerosol devices are known to those of skill in the art (See, e.g., Biesalski, U. S. Pat. Nos. 5,112,598; Biesalski, 5,556, 611, which are herein incorporated by reference). A cyclosporin derivative of this invention can also be formulated in rectal or vaginal compositions such as suppositories or retention enemas, e.g., containing conventional suppository bases such as cocoa butter or other glycerides. In addition to the formulations described previously, the cyclosporin derivatives of this invention can also be formulated as a depot preparation. Such long acting formulations can be administered by implantation (for example subcutaneously or intramuscularly) or by intramuscular injection. Thus, for example, the compounds can be formulated with suitable polymeric or hydrophobic materials (for example, as emulsion in acceptable oil) or ion exchange resins, or as sparingly soluble derivatives, for example, as a sparingly soluble salt. Suitable excipients (e.g., carriers and diluents) and other materials that can be used to provide mucosal dosage forms encompassed by this invention are well known to those skilled in the pharmaceutical arts, and depend on the particular site or method which a given pharmaceutical composition or dosage form will be administered. With that fact in mind, typical excipients include, but are not limited to, water, ethanol, ethylene glycol, propylene glycol, butane-1,3-diol, isopropyl myristate, isopropyl palmitate, mineral oil, and mixtures thereof, which are non-toxic and pharmaceutically acceptable.

The low toxicity of the conjugates allows a dosage in the range of 0.1-60 mg/kg body weight, whereas a dose of 5-15 mg/kg body weight is generally preferred.

The present invention furthermore refers to a method for increasing the activity of a drug compound in cells, comprising the steps of:

### a) transfecting cells with a conjugate according to the invention

### Transfection:

As cell model, Jurkat cells, a human T cell leukemia cell line (DSMZ Braunschweig, Germany), were used. Cells were cultured in RPMI1640 (Biochrom, Germany) supplemented with Glutamax I and 10% (v/v) heat-inactivated FCS at 37 °C in a humidified incubator. The transfection of the NFAT-luciferase reporter gene plasmid was performed with 5x10⁶ cells per sample in presence or absence of streptavidin by using an electroporation device (Amaxa, Cologne, Germany). After incubation with the compounds, stimulation with 50 nM PMA/2 µM ionomycin and incubation for 5 hours at 37 °C, cells were harvested and lysed. The level of expressed luciferase in cell lysates was determined at Bright-Glo^{™} Luciferase Assay System (Promega) according to manufacturer instructions.

### b) irradiating the cells of step a) with light radiation

### Irradiation:

Cells were irradiated in a 24-well plate by application of monochromatic light from an AlGaAs LED as light source. The light has a wavelength of 740 ± 30 nm and a radiant power of 15 mW (Roithner Lasertechnik, Vienna, Austria). The light source was installed 5 mm over the sample and was switched on fulfilling following irradiation sequence: incubation with compounds, 45 min irradiation, cell stimulation, 15 min break, 45 min irradiation, 15 min break, 45 min irradiation, 15 min break, 45 min irradiation, 15 min break, 45 min irradiation, and 15 min break.

### Description of the Figures.

**Figures 1A and 1B** show the structure-activity relationship of CsA and the chemical structures of conjugates comprising CsA derivatives. Compounds **1** and **2** in figure 1A represent conjugates according to the invention, whereas compound **3** is a two component conjugate comprising only a drug component A and a photoisomerizable group B. The orthogonal regulation of CaN enzyme activity is shown in figure 1B.
**Figure 2** refers to the photoisomerization of the CsA-AB-CsA conjugate **1** (as depicted in figure 1A) and the reverse thermal re-equilibration. Figure 2 shows the UV/Vis spectra of conjugate **1** in DMSO at 25°C. The samples are irradiated with light of either 370 nm (for 3 min) or 740 nm (for 45 min), either directly, or through a 1 cm path of human blood lysate.
**Figure 3** refers to the *trans* to cis photoisomerization of CsA-AB-CsA (compound **1** as depicted in figure 1A) induced by near UV or near IR light and the thermal re-equilibration. Figure 3 represents an analytical HPLC analysis of compound **1** before irradiation with light, after irradiations with light of 740 nm for different time periods, and after kept in darkness overnight. The measurements have been performed with a C-8 reverse phase HPLC column, with a gradient from acetonitrile/water (50 % / 50 %) to 100 % of acetonitrile. Both irradiations and HPLC analyses are performed at room temperature.

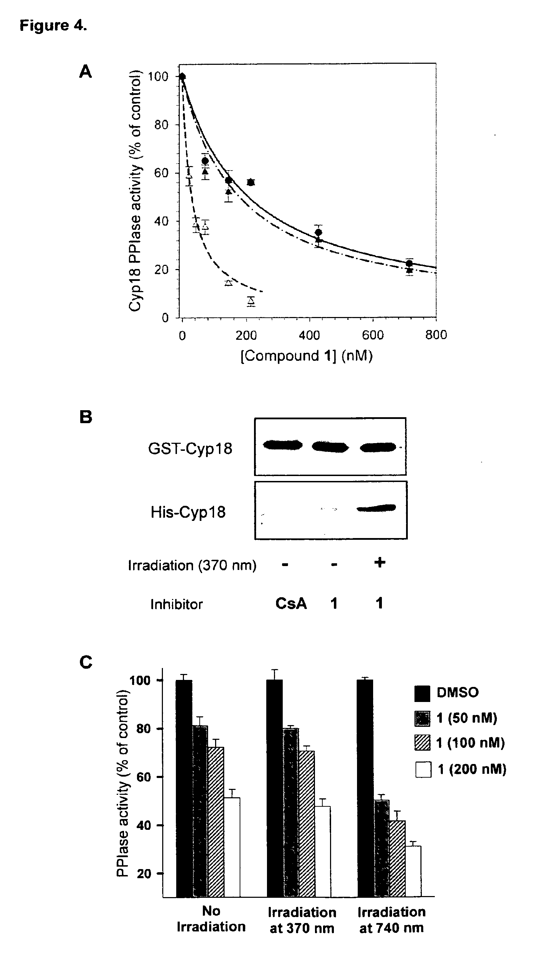

**Figures 4A, 4B and 4C** refer to the photo-regulation (with either near-UV or near-IR light) of Cyp18 PPIase activity by cyclosporin A derivatives.

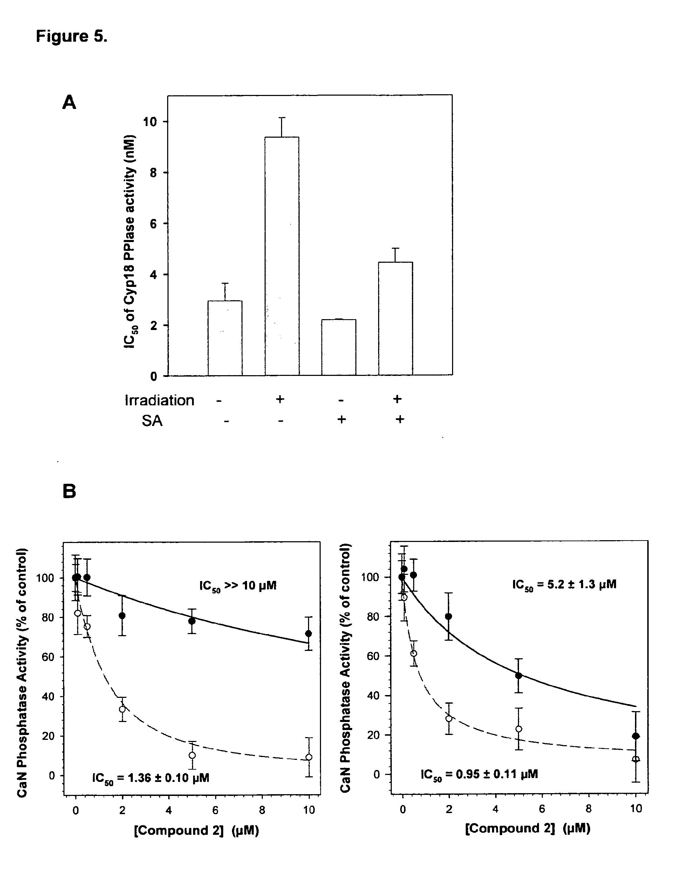

**Figures 5A and 5B** refer to the orthogonal regulation (with near-IR light irradiation and/or in presence of streptavidin, SA) of Cyp18 PPIase activity and calcineurin phosphatase activity by cyclosporin A derivatives.

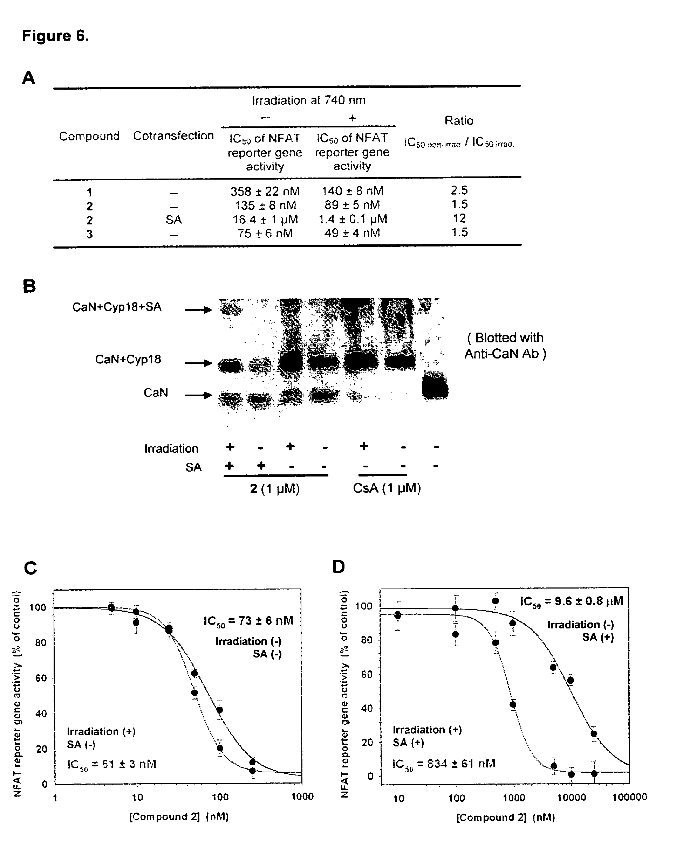

**Figures 6A - 6D** refer to the orthogonal regulation (with near-IR light irradiation and/or co-transfection of streptavidin) of transcriptional activation in activated T cells by of conjugates **1 - 3** as depicted in figure 1A.

### Examples

### Example 1. Preparation of the CsA-AB-CsA conjugate

A solution of 60 mg [D-Ser]⁸ CsA, tert-butyl bromoacetate (20 mg) and 5 mg benzyltriethylammonium chloride in 1 ml CH₂Cl₂ was stirred vigorously for two hours at room temperature with 2 ml 30 % NaOH aqueous solution. The reaction was then diluted with 10 ml water and extracted with ether twice. The organic layer was dried with Na₂SO₄. Without separation, the product was added to 60 mM KOH methanol solution and stirred at room temperature for 3 hours. Acetic acid was added. Most of the acetic acid and methanol were evaporated. Ethyl acetate was added and the mixture was washed with water. The organic layer is separated, dried with Na₂SO₄, evaporated and separated with RP HPLC (94%). 50 mg [O-carboxymethyl D-Ser]⁸ CsA and 0.5 equivalent 4,4'-Diaminoazobenzene were dissolved in 3 ml DMF. 3 equivalent of PyBOP ((Benzotriazol-1-yloxy)tripyrrolidinophosphonium hexafluorophosphate) and 5 equivalent of DIPEA (N-Ethyl-N,N-diisopropylamine) were added and the reaction mixture was stirred at room temperature over night. DMF was then removed under vacuum and ethyl acetate was added. The organic phase was washed with water 3 times and dried with Na₂SO₄ and evaporated and separated with RP HPLC (90 %).

### Example 2. Preparation of the CsA-AB-Biotin conjugate

50 mg [O-carboxymethyl D-Ser]⁸ CsA and 3 equivalent 4,4'-Diaminoazobenzene were dissolved in 3 ml DMF. 3 equivalent of PyBOP and 5 equivalent of DIPEA were added and the reaction mixture was stirred at room temperature over night. DMF was then removed and the conjugate of CsA and 4,4'-Diaminoazobenzene was separated with RP HPLC (85 %). The conjugate in 1ml DMF was added into a solution of biotin in 2 ml DMSO. 3 equivalent of PyBOP and 10 equivalent of DIPEA were added and the reaction mixture was stirred at room temperature over night. DMF was removed under vacuum and ethyl acetate was added. The organic phase was washed 3 times with 1 mM HCl and 3 times with water, and dried with Na₂SO₄. The ethyl acetate was removed under vacuum and the product CsA-AB-Biotin was separated by RP HPLC (80 %).

### Example 3. Photo-isomerization of CsA-AB-CsA and the reverse thermal re-equilibration.

Figure 2 shows that CsA-AB-CsA undergoes *trans* to *cis* photoisomerization under the irradiation through either 1-photon or 2-photon mechanism, and the resulting *cis* conformers possess completely different UV/Vis spectra from their corresponding *trans* counterparts. Moreover, the sample of blood lysate is transparent to light of 740 nm, but not to that of 370 nm. Therefore, only the light of 740 nm can pass the blood lysate sample to induce the photoisomerization. In the insert of Figure **2,** the UV time course at 370 nm of CsA-AB-CsA after irradiation shows that the *cis* to *trans* thermal isomerization is very slow, and the photoswitched cis conformer possesses a half-life of 3 hours at 25 °C.

### Example 4. The trans to cis photo-isomerization of CsA-AB-CsA induced by near UV or near IR light and the thermal re-equilibration.

Under irradiations of same intensities (2 mW for both 370 and 740 nm light), 2-photon photo-isomerization is slower than the 1-photon reaction, as analyzed by HPLC (Figure **3**). For example, while photo-stationary equilibrium of azobenzene (AB) derivatives (*cis*/*trans* ≈ 95/5) is reached within 3 min under irradiation at 370 nm, 45 min are required for completing the same reaction at 740 nm.

### Example 5. Enzymatic analysis of the inhibition of cyclophilin PPIase activity by the conjugates.

The PPIase activity was measured at 7 °C with a protease coupled assay on a Hewlett-Packard 8452a diode array spectrophotometer (Fischer et al., 1984). To 1220 µl Hepes buffer pH 7.8, Cyp18 and inhibitor of desired concentration are added. After 10 min of incubation, protease and substrate were added. The final substrate concentration is 40 µM in 1280 µl. The *cis*/*trans* isomerization was monitor at 390 nm with 510 nm as a reference. The rate is calculated according to first order reaction. Recombinant hCyp18 PPIase activity was measured with the peptide Suc-Ala-Phe-Pro-Phe-NH-Np as substrate. The substrate was dissolved in DMSO (10 mg/ml). α-Chymotrypsin was used as auxiliary protease. The residual enzymatic activity was plotted versus the inhibitor concentration and used for calculating the inhibitor constants Kᵢ as described before (Schutkowski et al., 1995).

### Example 6. Enzymatic analysis of the inhibition of calcineurin phosphatase activity by the conjugates.

The biotinylated and non-biotinylated RII peptides were phosphorylated by PKA (Baumgrass et al., 2004). The reaction mixture containing 700 µM peptide, 0.2 µM cAMP, 100 µCi[γ-³³P]ATP and 10 kU PKA in a final volume of 100 µl in the buffer (containing 20 mM MES, pH 6.5, 0.4 mM EDTA, 0.2 mM EGTA, 50 µM CaCl₂ and 2 mM MgCl₂) was incubated for one hour at 30 °C. The phosphorylated peptides were separated from unincorporated labeled ATP by 1 ml RP-C2 clean up extraction column (Amchro, Sulzbach, Germany). The eluted peptide (with 70% acetonitrile) was lyophilized and reconstituted with de-ionized water.

The scintillation proximity concept has been applied for measuring calcineurin activity using ScintiPlate wells coated with streptavidin. Pre-incubation of calmodulin (50 nM), calcineurin (1.32 nM) and inhibitors of desired concentrations in the assay buffer (40 mM Tris/HCl, pH 7.5; 100 mM NaCl, 6 mM MgCl₂, 0.5 mM DTT, 1 mM CaCl₂, 0.1 mg/ml BSA) was carried out for 30 min at 30 °C in a 96-well microtiter plate (Costar, Bodenheim, Germany). Biotinylated [γ-³³P] RII peptide was added to a final concentration of 100 nM and the total assay volume is 100 µl. After incubation at 30 °C for 30 min, 90 µl of the reaction mixture was transferred to the ScintiPlate well coated with streptavidin. The streptavidin immobilizes the biotinylated RII peptide on the wells. After 20 min at 22 °C the well was washed once with water and the RII associated [γ-³³P] was measured in the top-counter MicroBeta (Wallac, Turku, Finland).

### Example 7. An orthogonal regulation, by using light irradiation and adding assistant protein, of the inhibitory efficacies of CsA-AB-CsA to the PPIase activity of cyclophilin.

As shown in figure **4a****,** Inhibition of Cyp18 PPIase activity by non-irradiated CsA-AB-CsA (solid line, closed cycles), irradiated CsA-AB-CsA (dash, open triangles), and irradiated CsA-AB-CsA kept in darkness overnight (dash-dot, closed triangles). PPIase activities are measured with a protease coupled assay, using Suc-Ala-Phe-Pro-Phe-NH-Np as substrate and chymotrypsin as auxiliary protease, in 35 mM HEPES buffer pH 7.8, at 7 °C. Cyp18 concentration is 2.5 nM. A CsA derivative-free DMSO solution has been used as control. Irradiations have been performed with light of 740 nm for 45 min. As shown in Figure **5a****,** before adding into the solution of Cyp18 for inhibition analysis, CsA-AB-Biotin was pre-incubated with streptavidin and/or irradiated with light (740 nm). The concentrations of SA (calculated as monomer) are 4-equivalent to those of CsA-AB-Biotin. PPIase activities are measured with a protease coupled assay, using Suc-Ala-Phe-Pro-Phe-NH-Np as substrate and chymotrypsin as auxiliary protease, in 35 mM HEPES buffer pH 7.8, at 7 °C. Cyp18 concentration is 2.5 nM. Irradiations have been performed with light of 740 nm for 45 min.

The irradiated CsA-AB-CsA in cis exhibits a 7-fold increase of inhibitory potency towards the PPIase activity of Cyp18 (IC₅₀ = 30 ± 3 nM), as compared to the non-irradiated sample in *trans* (IC₅₀ = 206 ± 22 nM)(Figure **2A**). *Cis*-CsA-AB-CsA undergoes thermal cis to *trans* isomerization and the Cyp18 inhibitory profile has been reversed after keeping the irradiated sample in darkness overnight at room temperature. The samples in *cis* prepared by irradiation at either 370 nm or 740 nm are not distinguishable from each other in both HPLC and Cyp18 inhibition analyses.

Applying two orthogonal conditions (irradiation at 370 nm or 740 nm and addition of SA) on CsA-AB-Biotin results in four different states. Uncomplexed CsA-AB-Biotin and binary SA/CsA-AB-Biotin complex in either *trans* or photoswitched *cis* produce distinct inhibitory profiles on Cyp18 PPIase activity. While each *cis* conformer is more potent than the *trans* counterpart, SA bindings decrease the inhibitory efficiencies of both *cis-*CsA-AB-Biotin and *trans*-CsA-AB-Biotin. In the absence of SA, the difference of activity between the *cis* CsA-AB-Biotin (IC₅₀ = 2.21 ± 0.02 nM) and *trans* CsA-AB-Biotin (IC₅₀ = 2.96 ± 0.68 nM) is smaller than that between the *cis* (IC₅₀ = 4.45 ± 0.55 nM) and *trans* (IC₅₀ = 9.37 ± 0.75 nM) isomers bound to the borrowed protein SA. Although a moderate loss of activity is caused by the binding of the ligand CsA-AB-Biotin to the borrowed protein SA, the *cis* SA/CsA-AB-Biotin exerts full inhibition at reasonably low concentrations.

### Example 8. Analysis of the binary Cyp18/CsA-AB-CsA and ternary Cyp18/CsA-AB-CSA/Cyp18 complexes.

As shown in Figure **4b****,** coprecipitation of His₄-Cyp18 with GST-Cyp18-matrix (on glutathione-Sepharose) in the presence of non-irradiated CsA-AB-CsA, irradiated CsA-AB-CsA, and CsA. The proteins were eluted by boiling with sample buffer and separated by SDS-PAGE, transferred onto nitrocellulose membrane and probed with anti-His and anti-GST antibodies.

To distinguish the first and second binding events of two Cyp18 molecules to CsA-AB-CsA, coprecipitation experiments of an N-terminal His-tagged Cyp18 (His₄-Cyp18) with a GST-Cyp18 fusion protein in the presence of non-irradiated CsA-AB-CsA (*trans*-CsA-AB-CsA), irradiated CsA-AB-CsA (*cis*-CsA-AB-CsA), or CsA (as negative control), have been performed (Figure **4b**). His₄-Cyp18 is coprecipitated together with GST-Cyp18 by glutathione-Sepharose in the presence of photoswitched *cis-*CsA-AB-CsA, but not in the presence of CsA, while only a minor amount of His₄-Cyp18 has been detected in the presence of *trans*-CsA-AB-CsA. Furthermore, GST-Cyp18 binds to **cis**- and *trans* CsA-AB-CsA with similar potencies (data not shown), as accessed by analyzing the GST-Cyp18 bound CsA-AB-CsA by using HPLC. These results demonstrated that the 7-fold increase of Cyp18 inhibition by photoswitched CsA-AB-CsA is mainly contributed by a stabilized Cyp18/*cis*-CsA-AB-CsA/Cyp18 tertiary complex, as compared with the *trans* counterpart.

### Example 9. Enzymatic analysis and photoswitching effect of near UV and near IR lights on the inhibition of PPIase activity in blood lysate with the conjugates.

Blood lysates treated with CsA-AB-CsA or DMSO as negative control are kept in darkness or irradiated with light of 370 nm (for 3 min) or 740 nm (for 45 min). PPIase activities are measured with a protease coupled assay (Fischer et al., 1984), using Suc-Ala-Phe-Pro-Phe-NH-Np as substrate and chymotrypsin as auxiliary protease, in 35 mM HEPES buffer pH 7.8, at 7 °C.

In order to demonstrate the advantage of 2-photon photo-isomerization for potential biological and medical applications, we measured the inhibition of endogenous cyclophilins in blood lysate by CsA-AB-CsA, with or without irradiation (at either 370 nm or 740 nm, Figure **4c**). Cyp18 is the most abundant cytosolic cyclophilin and major receptor for CsA. Because the absorption of near-UV by blood lysate is much higher than that of near-IR, irradiation at 370 nm causes a little change of endogenous PPIase activity, whereas the light of 740 nm induces a remarkable decrease of PPIase activity, due to the production of photoswitched *cis* CsA-AB-CsA that possesses higher inhibitory potency. The change is not as big as that observed in the Cyp18 assay, since CsA analogues inhibit cyclophilins specifically, without affecting other families of PPIases in the blood lysate.

### Example 10. An orthogonal regulation, by using light irradiation and adding assistant protein, of the inhibitory efficacies by CsA-AB-Biotin on the phosphatase activity of calcineurin.

As shown in Figure **5b****,** before adding into the solution of calcineurin for inhibition analysis, CsA-AB-Biotin was pre-incubated with streptavidin and/or irradiated with light (740 nm). The concentrations of SA (calculated as monomer) are 4-equivalent to those of CsA-AB-Biotin. CaN phosphatase activities are measured with [³³P]labeled phosphocasein as substrate at 30 °C, in the presence of 10 µM of irradiated (right) or non-irradiated (left) CsA-AB-Biotin and Cyp18 of desired concentrations, with (black) or without (red) SA. The inhibition concentration designates the concentration of Cyp18/CsA-AB-Biotin complex. CaN concentration is 3.5 nM. A CsA derivative-free DMSO solution has been used as control. The result has been confirmed in three independent experiments. The IC₅₀ of the non-irradiated CsA-AB-Biotin in the presence of SA is too high to be determined precisely, due to the poor solubility of compound CsA-AB-Biotin above 10 µM.

In CaN inhibition assays, the 4 different states, Cyp18/CsA-AB-Biotin in *cis* and *trans,* and Cyp18/CsA-AB-Biotin/SA in *cis* and *trans,* exhibit dramatically different inhibitory potencies (Figure **5b**). While binary Cyp18/CsA-AB-Biotin in *cis* is most efficient in CaN inhibition (IC₅₀ = 0.95 ± 0.11 µM), the *trans* form of Cyp18/CsA-AB-Biotin/SA complex shows only 30 % inhibition at concentration as high as 10 µM. Eventually, the crystal structures of Cyp18/CsA/Ca complex indicate that the recruitment of a large molecule via CsA residue 8 could cause a great steric hindrance and prevent the Cyp18/CsA-AB-Biotin/SA tertiary complex from binding to CaN. In fact, the augmentation of difference between the reversibly switchable *cis* and *trans* resulted from borrowing SA is very remarkable in the CaN inhibition analysis, whereas the simple photoswitchable side chains on reside 8 of CsA, such as those of CsA-AB-Biotin in the absence of SA or derivative CsA-AB (data not shown), have shown that the conformational difference of AB between the *cis* and *trans* forms could results in only a minor change of CaN inhibition. Different from its CaN inactive *trans* counterpart, the photoswitched Cyp18/CsA-AB-Biotin/SA in *cis* exhibits moderate inhibitory effect on CaN (IC₅₀ = 5.17 ± 1.28 µM). The different topological arrangements of Cyp18 and SA within the tertiary Cyp18/CsA-AB-Biotin/SA associated with *cis* and *trans* conformations of AB group could account for the different accessibilities of *cis* and *trans* complexes to CaN. Our data has demonstrated that borrowing protein could cause not only steric hindrance diminishing protein/ligand interactions, but also an enlarged difference of steric hindrance between two inter-convertible conformers.

### Example 11. An in vitro orthogonal regulation, by using light irradiation and the presence of assistant protein, of the inhibitory efficacies by CsA-AB-CsA, CsA-AB-Biotin, and CsA-AB on T cell transcriptional activation.

As shown in Figure **6a****,** Jurkat T cells were transfected with an NFAT-luciferase reporter plasmid. Cells were pretreated with irradiated (740 nm) or non-irradiated CsA-AB-CsA, CsA-AB-Biotin, or CsA-AB and stimulated with PMA/ionomycin for 5 h, or cells were additionally cotransfected with SA, pretreated with irradiated or non-irradiated CsA-AB-Biotin and stimulated with PMA/ionomycin for 5 h. After cell lysis, the level of the extracted luciferase activity was determined. Data are expressed as percentages of luminescence activity relative to the stimulated control in absence of inhibitor.

To demonstrate that different CaN inhibitory efficacies between *cis* and *trans* conformers of the CsA derivatives conjugated to AB could lead to distinct signaling patterns in activated T cells, we have measured their suppressive effects with an NFAT reporter gene assay, because the dephosphorylation and activation of NFAT are mediated by CaN phosphatase activity (Figure **6**). Without borrowing a protein through high affinity interaction, both CsA derivative CsA-AB-Biotin and CsA-AB exhibit minor difference between the *cis* and *trans* conformers in suppressing NFAT transcriptional activation, with a *trans*/*cis* difference of activity of 1.5. Similar difference of activity of 1.4 and 1.5 have been also observed in CaN inhibition assay by CsA-AB-Biotin (in the absence of SA) and CsA-AB, respectively.

Given that Cyp18 is an abundant cytosolic protein in T cells, some molecules of CsA derivative CsA-AB-CsA are expected to bind to two Cyp18 and to form tertiary complex in cells. As discussed above, because it is difficult to determine the ratio of binary Cyp18/CsA-AB-CsA to tertiary Cyp18/CsA-AB-CsA/Cyp18, the contribution from the formation of tertiary complex cannot be evaluated exactly. Nevertheless, as compared with CsA derivative CsA-AB-Biotin and CsA-AB (Figure **6a****),** the 2.5-fold *trans*/*cis* difference of activity of compound CsA-AB-CsA indicates an augmentation caused by the recruitment of a second Cyp18 as a borrowed protein. Interestingly, the observation from cell-based analysis is in good agreement with the difference of activity obtained from CaN inhibition assay.

Different from CsA-AB-CsA, the 3 interactions (borrowing protein SA, Cyp18 binding, and CaN inhibition) can be clearly distinguished by using CsA derivative CsA-AB-Biotin. In cells transfected with SA (Figure **6a****),** photoswitched *cis*-CsA-AB-Biotin exhibits a remarkably higher suppressive activity, as compared to that of *trans*-CsA-AB-Biotin, resembling the difference between the *cis* and *trans* Cyp18/CsA-AB-Biotin/SA in CaN phosphatase inhibition assay (Figure **5b**). Borrowing protein through the strong binding affinity of SA to the biotin moiety in CsA-AB-Biotin leads to a high *trans*/*cis* difference of activity of 12 (Figure **6a****).** In contrast, in the absence of cellular SA, compound CsA-AB-Biotin gives a low *trans*/*cis* difference of activity of 1.5, similar to that of CsA-AB-Biotin.

### Example 12. Analysis of the 4-component Cyp18/CsA-AB-Biotin/SA/calcineurin complexes.

As shown in Figure **6b****,** Jurkat cells were transfected with SA or mock and treated with irradiated or non-irradiated compound CsA-AB-CsA. Cells were lysed and the clear supernatants were incubated with calmodulin sepharose. Cross-linking reagent BS³ was added to the EDTA eluates. After TCA precipitation, the samples were separated by SDS-PAGE and probed with anti-CaN antibody and detected by enhanced chemiluminescent reaction.

In order to demonstrate that not only functional but also physical interactions between CaN and Cyp18 are involved in CsA-AB-Biotin treated cells and regulated by light irradiation and the presence of SA, we performed a cross-linking experiment (Figure **6b**). In the absence of SA, both CsA and photoswitched *cis*-CsA-AB-Biotin induced an intensive cross-linking between CaN and Cyp18. The weakest cross-linking effect has been observed from cells transfected with SA and treated with non-irradiated *trans*-CsA-AB-Biotin. Cross-linked CaN-Cyp18-SA has been detected only in sample treated with *cis*-CsA-AB-Biotin, indicating that the Cyp18-*trans*-CsA-AB-Biotin-SA complex does not interact with CaN. These results correlate well with the phosphatase (Figure **5b****)** and reporter gene (Figure **6a****)** activities obtained from the combinations of 2 orthogonal conditions.

### Example 13. An in situ orthogonal regulation, by using light irradiation and the presence of assistant protein, of the inhibitory efficacies by CsA-AB-Biotin on T cell transcriptional activation.

As shown in Figure **6c** and **6d****,** Jurkat T cells were transfected with an NFAT-luciferase reporter plasmid. Cells without (C) or with (D) additional SA cotransfection were pretreated with non-irradiated CsA-AB-Biotin and the cells were irradiated with light of 740 nm (red) or kept in dark (black) for 1 hour before PMA/ionomycin stimulation. After cell lysis, the level of the extracted luciferase activity was determined. Data are expressed as percentages of luminescence activity relative to the stimulated control in absence of inhibitor.

The effects of IR light (740 nm) induced 2-photon photo-isomerization on cells pretreated with CsA-AB-Biotin has been evaluated with the NFAT reporter gene assay (figure **6c** and **6d**). In cells without SA transfection, the enhancement of inhibition induced by IR irradiation was minor (1.5-fold, figure **6c**). In contrast, irradiation caused an 11.5-fold increase of inhibitory efficacy in the cells transfected with SA (figure **6d**). Like the experiments using preformed *cis* and *trans* CsA-AB-Biotin (figure **6a****),** IR light of 740 nm is able to photo-isomerize an individual bond (AB in CsA-AB-Biotin) selectively in cells and modulates the transcriptional activation of activated T cells *in situ.* Moreover, a dramatic augmentation of the photo-switchable effect has been achieved in the presence of cellular SA.

### Example 14. Interpretation of the experimental results.

Although *trans* to *cis* photo-isomerization of a CsA-AB conjugates can result in switched inhibitory potencies on Cyp18 and CaN, the use of a drug component and a photoisomerizable group can only account for a moderate difference between the *cis* and *trans* photo-isomers. By providing an additional small molecule protein binding moiety, which is bonded to the azobenzene, the topological effect induced by azobenzene isomerization is increased by the interaction between the small molecule protein binding moiety and the enzyme (figure 1B).

The protein-borrowing approach causes an increase of the influence of a local conformational change on the steric arrangements of the components within the complex as well as the hindrance in target protein binding and inhibition. If e.g. a system of bifunctional CsA derivatives comprising biotin as a small molecule protein binding moiety (figures 1A and 1B) is used, the CsA component as well as the biotin will interact with CaN, which leads to a significant difference between the properties of the *cis* and *trans* isomers. The effect the conjugates **1** and **2** of azobenzene isomerization on Cyp18 inhibition and Cyp18 dependent CaN inhibition has been investigated. As shown in figure 1, CsA and a second ligand are linked by azobenzene in order to achieve maximal geometrical difference between the 2 conformers of the protein/ligand/protein complexes, which results in distinct quaternary complex formations with and inhibitions of CaN.

The CsA-AB-biotin conjugate is of particular interest because: on the one hand, the introduction of biotin leads to a CaN inhibiting system, whose potency is sensitive not only to light irradiation, but also to the presence of biotin binding proteins, such as streptavidin (SA); on the other hand, the high affinity between biotin and SA allows to dissect their interaction from other binding events in cells as well as in enzyme activity analysis *in vitro* (as discussed below).

It has been found that the irradiated CsA-AB-CsA in *cis* conformation exhibits a 7-fold increase of inhibitory potency towards the PPIase activity of Cyp18 (IC₅₀ = 30 ± 3 nM), as compared to the non-irradiated sample in *trans* conformation (IC₅₀ = 206 ± 22 nM). *Cis*-CsA-AB-CsA undergoes thermal *cis* to *trans* isomerization and the Cyp18 inhibitory profile has been reversed after keeping the irradiated sample in darkness overnight at room temperature. The samples in *cis* prepared by irradiation at either 370 nm or 740 nm are not distinguishable from each other in both HPLC and Cyp18 inhibition analyses. Because the *cis* conformers give higher absorption coefficients than the *trans* conformers above 420 nm, the *cis* to *trans* thermal isomerization can be accelerated by irradiating at 450 nm. Using a CaN phosphatase activity assay, the CaN inhibitory efficiencies of *cis*-CsA-AB-CsA and *trans*-CsA-AB-CsA have been measured in the presence of Cyp18. A significant increase of CaN inhibition has been observed upon light irradiation.

To distinguish the first and second binding events of two Cyp18 molecules to CsA-AB-CsA, coprecipitation experiments of an N-terminal His-tagged Cyp18 (His₄-Cyp18) with a GST-Cyp18 fusion protein in the presence of non-irradiated CsA-AB-CsA (*trans*-CsA-AB-CsA), irradiated CsA-AB-CsA (*cis*-CsA-AB-CsA), or CsA (as negative control), have been performed. His₄-Cyp18 is coprecipitated together with GST-Cyp18 by glutathione-Sepharose in the presence of photoswitched *cis*-CsA-AB-CsA, but not in the presence of CsA, while only a minor amount of His4-Cyp18 has been detected in the presence of *trans*-CsA-AB-CsA. Furthermore, GST-Cyp18 binds to *cis*- and *trans* CsA-AB-CsA with similar potencies (data not shown), as accessed by analyzing the GST-Cyp18 bound CsA-AB-CsA by using HPLC. These results demonstrate that the 7-fold increase of Cyp18 inhibition by photoswitched CsA-AB-CsA is mainly contributed by a stabilized Cyp18/*cis*-CsA-AB-CsA/Cyp18 tertiary complex, as compared with the *trans* counterpart.

In order to demonstrate the advantage of 2-photon photo-isomerization for potential biological and medical applications, the inhibition of endogenous cyclophilins in blood lysate by CsA-AB-CsA, with or without irradiation (at either 370 nm or 740 nm) has been measured (figure 2). Cyp18 is the most abundant cytosolic cyclophilin and major receptor for CsA. Because the absorption of near-UV by blood lysate is much higher than that of near-IR, irradiation at 370 nm causes a little change of endogenous PPIase activity, whereas the light of 740 nm induces a remarkable decrease of PPIase activity, due to the production of photoswitched *cis* CsA-AB-CsA that possesses higher inhibitory potency. The change is not as big as that observed in the Cyp18 assay, since CsA analogues inhibit cyclophilins specifically, without affecting other families of PPIases in the blood lysate.

Furthermore, CsA-AB-Biotin has been designed. Since the affinity of biotin to SA (K_{d} ∼ 10 fM) is much higher than that between Cyp18 and CsA (K_{d} = 9 nM), and the CaN inhibition (IC₅₀ = 100 nM) by CsA is mediated through a gain-of-function mechanism upon Cyp18 binding, the sequential interactions and resulted regulations are one directional in the order: SA/biotin binding → Cyp18/CsA interaction → CaN inhibition.

Applying two orthogonal conditions (irradiation at 370 nm or 740 nm and addition of SA) on CsA-AB-Biotin results in four different states (figure 1). Uncomplexed CsA-AB-Biotin and binary SA/CsA-AB-Biotin complex in either *trans* or photoswitched *cis* produce distinct inhibitory profiles on Cyp18 PPIase activity. While each *cis* conformer is more potent than the *trans* counterpart, SA bindings decrease the inhibitory efficiencies of both *cis*-CsA-AB-Biotin and *trans-*CsA-AB-Biotin. In the absence of SA, the difference of activity between the *cis* CsA-AB-Biotin (IC₅₀ = 2.21 ± 0.02 nM) and *trans* CsA-AB-Biotin (IC₅₀ = 2.96 ± 0.68 nM) is smaller than that between the *cis* (IC₅₀ = 4.45 ± 0.55 nM) and *trans* (IC₅₀ = 9.37 ± 0.75 nM) isomers bound to the borrowed protein SA. Although a moderate loss of activity is caused by the binding of the ligand CsA-AB-Biotin to the borrowed protein SA, the *cis* SA/CsA-AB-Biotin exerts full inhibition at reasonably low concentrations.

In CaN inhibition assays, the 4 different states, Cyp18/CsA-AB-Biotin in *cis* and *trans,* and Cyp18/CsA-AB-Biotin/SA in *cis* and *trans,* exhibit dramatically different inhibitory potencies. While binary Cyp18/CsA-AB-Biotin in *cis* is most efficient in CaN inhibition (IC₅₀ = 0.95 ± 0.11 µM), the *trans* form of Cyp18/CsA-AB-Biotin/SA complex shows only 30 % inhibition at concentration as high as 10 µM. The protein-borrowing strategy is suitable in order to enhance the affinities of inhibitors to their targets, by converting a single ligand into a bifunctional dimer and using the attached ligand to recruit a presenter protein. However, it has also been speculated that the most potential use of a borrowed protein may be for abolishing interaction. As for multicomponent complex formation, to recruit macromolecule for raising steric hindrance would be expected to cause more remarkable influence than to create an energetically favorable protein-protein interface. Eventually, the crystal structures of Cyp18/CsA/CaN complex indicate that the recruitment of a large molecule via CsA residue 8 could cause a great steric hindrance and prevent the Cyp18/CsA-AB-Biotin/SA tertiary complex from binding to CaN. In fact, the augmentation of difference between the reversibly switchable *cis* and *trans* conformations resulted from borrowing SA is very remarkable in the CaN inhibition analysis, whereas the simple photoswitchable side chains on reside 8 of CsA, such as those of CsA-AB-Biotin in the absence of SA or derivative CsA-AB, have shown that the conformational difference of azobenzene between the *cis* and *trans* forms could results in only a minor change of CaN inhibition. Different from its CaN inactive *trans* counterpart, the photoswitched Cyp18/CsA-AB-Biotin/SA in *cis* exhibits moderate inhibitory effect on CaN (IC₅₀ = 5.17 ± 1.28 µM). The different topological arrangements of Cyp18 and SA within the tertiary Cyp18/CsA-AB-Biotin/SA associated with *cis* and *trans* conformations of azobenzene group could account for the different accessibilities of *cis* and *trans* complexes to CaN.

To demonstrate that different CaN inhibitory efficacies between *cis* and *trans* conformers of the CsA derivatives conjugated to AB lead to distinct signaling patterns in activated T cells, their suppressive effects have been measured with a luciferase-coupled NFAT reporter gene assay (examples 11 and 13), because the dephosphorylation and activation of NFAT are mediated by CaN phosphatase activity. Without borrowing a protein through high affinity interaction, both CsA derivative CsA-AB-Biotin and CsA-AB exhibit minor difference between the *cis* and *trans* conformers in suppressing NFAT transcriptional activation, with a *trans*/*cis* difference of activity of 1.5. Similar differences of activity of 1.4 and 1.5 have been also observed in CaN inhibition assay by CsA-AB-Biotin (in the absence of SA) and CsA-AB, respectively.

Given that Cyp18 is an abundant cytosolic protein in T cells, some molecules of CsA derivative CsA-AB-CsA are expected to bind two Cyp18 molecules and to form tertiary complex in cells. As discussed above, because it is difficult to determine the ratio of binary Cyp18/CsA-AB-CsA to tertiary Cyp18/CsA-AB-CsA/Cyp18, the contribution from the formation of tertiary complex cannot be evaluated exactly. Nevertheless, as compared with CsA derivative CsA-AB-Biotin and CsA-AB, the 2.5-fold *trans*/*cis* difference of activity of compound CsA-AB-CsA indicates an augmentation caused by the recruitment of a second Cyp18 as a borrowed protein. Interestingly, the observation from cell-based analysis is in good agreement with the difference of activity obtained from CaN inhibition assay.

Different from CsA-AB-CsA, the 3 interactions (borrowing protein SA, Cyp18 binding, and CaN inhibition) can be clearly distinguished by using CsA derivative CsA-AB-Biotin. In cells transfected with SA, photoswitched *cis*-CsA-AB-Biotin exhibits a remarkably higher suppressive activity, as compared to that of *trans*-CsA-AB-Biotin, resembling the difference between the *cis* and *trans* Cyp18/CsA-AB-Biotin/SA in CaN phosphatase inhibition assay. The protein borrowing through the strong binding affinity of SA to the biotin moiety in CsA-AB-Biotin leads to a high *trans*/*cis* difference of activity of 12. In contrast, in the absence of cellular SA, compound CsA-AB-Biotin gives a low *trans*/*cis* difference of activity of 1.5, similar to that of CsA-AB-Biotin. In order to demonstrate that not only functional but also physical interactions between CaN and Cyp18 are involved in CsA-AB-Biotin treated cells and regulated by light irradiation and the presence of SA, a cross-linking experiment has been performed. In the absence of SA, both CsA and photoswitched *cis*-CsA-AB-Biotin induced an intensive cross-linking between CaN and Cyp18. The weakest cross-linking effect has been observed from cells transfected with SA and treated with non-irradiated *trans*-CsA-AB-Biotin. It has been found that cross-linked CaN-Cyp18-SA can only be detected in samples treated with *cis*-CsA-AB-Biotin, indicating that the Cyp18-*trans*-CsA-AB-Biotin-SA complex does not interact with CaN. These results correlate well with the phosphatase and reporter gene activities obtained from the combinations of 2 orthogonal conditions (figure 1).

The effects of IR light (740 nm) induced 2-photon photo-isomerization on cells pretreated with CsA-AB-Biotin have been evaluated with the NFAT reporter gene assay (example 11). In cells without SA transfection, the enhancement of inhibition induced by IR irradiation was minor (1.5-fold). In contrast, irradiation caused an 11.5-fold increase of inhibitory efficacy in the cells transfected with SA. Like the experiments using preformed *cis* and *trans* CsA-AB-Biotin, IR light of 740 nm is able to photo-isomerize an individual bond (AB in CsA-AB-Biotin) selectively in cells and modulates the transcriptional activation of activated T cells *in situ.* Moreover, a dramatic augmentation of the photo-switchable effect has been achieved in the presence of cellular SA.

### References

(1990). Remington's pharmaceutical sciences, 18th Ed. edn: Mack Pub. Co. (Easton, Pa.)).
Advani, R., Horwitz, S., Zelenetz, A., and Horning, S. J. (2007). Angioimmunoblastic T cell lymphoma: treatment experience with cyclosporine. Leuk Lymphoma 48, 521-525.
Ansel, H. C. (1985). Introduction to Pharmaceutical Dosage Forms: Lea. &. Febiger, Washington Square, Philadelphia, PA 19106.).
Baumgrass, R., Zhang, Y., Erdmann, F., Thiel, A., Weiwad, M., Radbruch, A., and Fischer, G. (2004). Substitution in position 3 of cyclosporin A abolishes the cyclophilin-mediated gain-of-function mechanism but not immunosuppression. J Biol Chem 279, 2470-2479.
Bednarski, P. J., Mackay, F. S., and Sadler, P. J. (2007). Photoactivatable platinum complexes. Anticancer Agents Med Chem 7, 75-93.
Berth-Jones, J. (2005). The use of ciclosporin in psoriasis. J Dermatolog Treat 16, 258-277.
Borel, J. F. (1990). Pharmacology of cyclosporine (sandimmune).. Pharmacol Rev 41, 259.
Briesewitz, R., Ray, G. T., Wandless, T. J., and Crabtree, G. R. (1999). Affinity modulation of small-molecule ligands by borrowing endogenous protein surfaces [see comments]. Proc Natl Acad Sci 96, 1953-1958.
Busauschina, A., Schnuelle, P., and van der Woude, F. J. (2004). Cyclosporine nephrotoxicity. Transplant Proc 36, 229S-233S.
Cahalan, M. D., Parker, I., Wei, S. H., and Miller, M. J. (2002). Two-photon tissue imaging: seeing the immune system in a fresh light. Nat Rev Immunol 2, 872-880.
Churikov, V. M., Lin, J. T., Wu, H. H., Lin, J. H., Huang, T. H., and Hsu, C. C. (2002). One- and two-photon induced molecular conformation change and reorientation and related third-order nonlinearities in phenylamine azo-dye polymer thin films. Opt Commun 209, 451-460.
Cruz, M. C., Del Poeta, M., Wang, P., Wenger, R., Zenke, G., Quesniaux, V. F., Movva, N. R., Perfect, J. R., Cardenas, M. E., and Heitman, J. (2000). Immunosuppressive and nonimmunosuppressive cyclosporine analogs are toxic to the opportunistic fungal pathogen Cryptococcus neoformans via cyclophilin-dependent inhibition of calcineurin. Antimicrob Agents Chemother 44, 143-149.
Dolmetsch, R. E., Xu, K., and Lewis, R. S. (1998). Calcium oscillations increase the efficiency and specificity of gene expression. Nature 392, 933-936.
Fischer, G. (1994). Peptidyl-prolyl cis/trans isomerases and their effectors. Angew Chem Int Ed Engl 33, 1415-1436.
Fischer, G., Bang, H., and Mech, C. (1984). Determination of enzymatic catalysis for the cis-trans-isomerization of peptide binding in proline-containing peptides. Biomed Biochim Acta 43, 1101.
Foxwell, B. M., Mackie, A., Ling, V., and Ryffel, B. (1989). Identification of the multidrug resistance-related P-glycoprotein as a cyclosporine binding protein. Mol Pharmacol 36, 543-546.
Gestwicki, J. E., Crabtree, G. R., and Graef, I. A. (2004). Harnessing chaperones to generate small-molecule inhibitors of amyloid beta aggregation. Science 306, 865-869.
Graef, I. A., Chen, F., and Crabtree, G. R. (2001). NFAT signaling in vertebrate development. Curr Opin Genet Dev 11, 505-512.
Gwack, Y., Feske, S., Srikanth, S., Hogan, P. G., and Rao, A. (2007). Signalling to transcription: store-operated Ca2+ entry and NFAT activation in lymphocytes. Cell Calcium 42, 145-156.
Ho, V. C. (2004). The use of ciclosporin in psoriasis: a clinical review. Br J Dermatol 150 Suppl 67, 1-10.
Huai, Q., Kim, H. Y., Liu, Y., Zhao, Y., Mondragon, A., Liu, J. O., and Ke, H. (2002). Crystal structure of calcineurin-cyclophilin-cyclosporin shows common but distinct recognition of immunophilin-drug complexes. Proc Natl Acad Sci 99, 12037-12042.
Jin, L., and Harrison, S. C. (2002). Crystal structure of human calcineurin complexed with cyclosporin A and human cyclophilin. Proc Natl Acad Sci 99, 13522.
Kitahara, K., and Kawai, S. (2007). Cyclosporine and tacrolimus for the treatment of rheumatoid arthritis. Curr Opin Rheumatol 19, 238-245.
Knight, V., Koshkina, N. V., Golunski, E., Roberts, L. E., and Gilbert, B. E. (2004). Cyclosporin a aerosol improves the anticancer effect of Paclitaxel aerosol in mice. Trans Am Clin Climatol Assoc 115, 395-404.
Liu, J., Farmer, J. D., Jr., Lane, W. S., Friedman, J., Weissman, I., and Schreiber, S. L. (1991). Calcineurin is a common target of cyclophilin-cyclosporin A and FKBP- FK506 complexes. Cell 66, 807-815.
Medyouf, H., Alcalde, H., Berthier, C., Guillemin, M. C., dos Santos, N. R., Janin, A., Decaudin, D., de The, H., and Ghysdael, J. (2007). Targeting calcineurin activation as a therapeutic strategy for T-cell acute lymphoblastic leukemia. Nat Med 13, 736-741.
Morgan, R. J., Jr., Synold, T. W., Gandara, D., Muggia, F., Scudder, S., Reed, E., Margolin, K., Raschko, J., Leong, L., Shibata, S., et al. (2007). Phase II trial of carboplatin and infusional cyclosporine with alpha-interferon in recurrent ovarian cancer: a California Cancer Consortium Trial. Int J Gynecol Cancer 17, 373-378.
Neri, D., and Bicknell, R. (2005). Tumour vascular targeting. Nat Rev Cancer 5, 436-446.
Odom, A., Del Poeta, M., Perfect, J., and Heitman, J. (1997). The immunosuppressant FK506 and its nonimmunosuppressive analog L-685,818 are toxic to Cryptococcus neoformans by inhibition of a common target protein. Antimicrob Agents Chemother 41, 156-161.
Oh, Y. L., Han, S. Y., Mun, K. H., Choi, H. J., Kim, H. Y., Hwang, E. A., Park, S. B., Kim, H. C., and Chang, E. J. (2006). Cyclosporine-induced apoptosis in osteosarcoma cells. Transplant Proc 38, 2237-2239.
Olbe, L., Carlsson, E., and Lindberg, P. (2003). A proton-pump inhibitor expedition: the case histories of omeprazole and esomeprazole. Nat Rev Drug Discov 2, 132-139.
Periyasamy, S., Warrier, M., Tillekeratne, M. P., Shou, W., and Sanchez, E. R. (2007). The immunophilin ligands cyclosporin A and FK506 suppress prostate cancer cell growth by androgen receptor-dependent and -independent mechanisms. Endocrinology 148, 4716-4726.
Renner, C., and Moroder, L. (2006). Azobenzene as conformational switch in model peptides. Chembiochem 7, 868-878.
Ryffel, B., Woerly, G., Rodriguez, C., and Foxwell, B. M. (1991). Identification of the multidrug resistance-related membrane glycoprotein as an acceptor for cyclosporine. J Recept Res 11, 675-686.
Scheinfeld, N. (2007). A review of deferasirox, bortezomib, dasatinib, and cyclosporine eye drops: possible uses and known side effects in cutaneous medicine. J Drugs Dermatol 6, 352-355.
Schon, M. P., Detmar, M., and Parker, C. M. (1997). Murine psoriasis-like disorder induced by naive CD4+ T cells. Nat Med 3, 183-188.
Schreiber, S. L., and Crabtree, G. R. (1992). The mechanism of action of cyclosporin A and FK506. Immunol Today 13, 136-142.
Schutkowski, M., Wollner, S., and Fischer, G. (1995). Inhibition of peptidyl-prolyl cis/trans isomerase activity by substrate analog structures: thioxo tetrapeptide-4-nitroanilides. Biochemistry 34, 13016-13026.
Serafini, D. M., and Schellhorn, H. E. (1999). Endonuclease III and endonuclease IV protect Escherichia coli from the lethal and mutagenic effects of near-UV irradiation. Can J Microbiol 45, 632-637.
Steinbach, W. J., Reedy, J. L., Cramer, R. A., Jr., Perfect, J. R., and Heitman, J. (2007). Harnessing calcineurin as a novel anti-infective agent against invasive fungal infections. Nat Rev Microbiol 5, 418-430.
Tunc, M., and Erbilen, E. (2006). Topical cyclosporine-a combined with mitomycin C for conjunctival and corneal squamous cell carcinoma. Am J Ophthalmol 142, 673-675.
Wilson, L. S., Reyes, C. M., Stolpman, M., Speckman, J., Allen, K., and Beney, J. (2002). The direct cost and incidence of systemic fungal infections. Value Health 5, 26-34.
Xia, C. Q., Liu, N., Miwa, G. T., and Gan, L. S. (2007). Interactions of cyclosporin a with breast cancer resistance protein. Drug Metab Dispos 35, 576-582.
Zhang, Y., Erdmann, F., Baumgrass, R., Schutkowski, M., and Fischer, G. (2005). Unexpected side chain effects at residue 8 of cyclosporin a derivatives allow photoswitching of immunosuppression. J Biol Chem 280, 4842-4850.
Zhou, X., Ren, A. M., Feng, J. K., Liu, X. J., Zhang, J. X., and Liu, J. Z. (2002). One- and two-photon absorption properties of novel multi-branched molecules. Phy Chem Chem Phy 4, 4346-4352.

## Claims

1. A drug conjugate of the general formula A-B-C, wherein A is a drug component, B is a photoisomerizable group and C is a small molecule protein binder.

2. Conjugate according to claim 1, wherein A is selected from the group consisting of cyclosporin A derivatives, FK506, rapamycin, pimecrolimus, everolimus, sanglifehrin, and derivatives thereof.

3. Conjugate according to claims 1 or 2, wherein B is selected from the group consisting of azobenzene, stilbene, spiropyrane, diarylethene, fulgide, and derivatives thereof.

4. Conjugate according to claims 1 - 3, wherein the small molecule protein binder C is selected from the group consisting of cyclosporin A derivatives, biotin, desthiobiotin, iminobiotin; cyclophilin binding ligands; FK506 and FKBP binding ligands; albumin binding ligands; the VIVIT peptide and derived sequences that bind to calcineurin; taxol, epothilone and their derivatives that bind to the αβ-subunit of tubulin; Strep-tag I, Strep-tag II and derived sequences that bind to streptavidin, avidin or their derivatives; a hapten or antigen that bind to monoclonal or polyclonal antibodies; poly-histidine-tag that binds to metal-containing complexes; metal/chelator-complexes that bind to poly-histidine-tag containing peptides or proteins; nucleic acids, nucleotides, and oligonucleotides that can be hybridized to other oligonucleotide sequences specifically, or can form a complex with nucleotide and oligonucleotide binding proteins.

5. Conjugate according to claims 3 or 4, wherein an azobenzene derivative is represented by formula **3:** 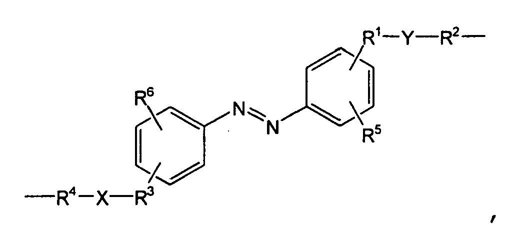 wherein
R¹, R², R³, R⁴ are independently selected from branched, un-branched or cyclic C₁ - C₁₀ hydrocarbyl groups;
X, Y are independently selected from O, S, -NR⁷-, -C(O)NR⁷-, -C(S)NR⁷-, -NR⁷(C(O)-, and -NR⁷C(S)-;
R₇ is H, methyl, ethyl, or propyl;
R⁵ and R⁶ are independently selected from H, F, Cl, Br, I, branched, un-branched or cyclic C₁ - C₁₀ hydrocarbyl groups, NO₂, CF₃, OR⁹, OCOR⁹, COOR⁹, CH₂OR⁹, CHO, COR⁹, NR⁹R¹⁰ and SR⁹; R⁹ is H and branched, un-branched or cyclic C₁ - C₁₀ hydrocarbyl groups.

6. Conjugate according to claims 3 or 4, wherein a stilbene derivative is represented by formula **4:** 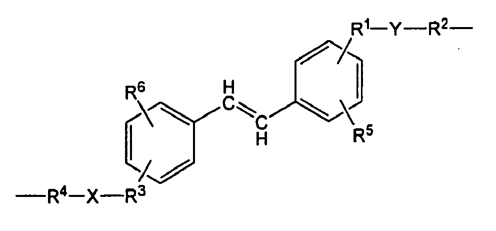
R¹, R², R³, R⁴ are independently selected from branched, un-branched or cyclic C₁ - C₁₀ hydrocarbyl groups;
X, Y are independently selected from O, S, -NR⁷-, -C(O)NR⁷-, -C(S)NR⁷-, -NR⁷(C(O)-, and -NR⁷C(S)-;
R⁵ and R⁶ are independently selected from H, F, Cl, Br, I, branched, un-branched or cyclic C₁ - C₁₀ hydrocarbyl groups, NO₂, CF₃, OR⁹, OOR⁹, COOR⁹, CH₂OR⁹, CHO, COR⁹, NR⁹R¹⁰ and SR⁹; R⁹ is H or branched, un-branched or cyclic C₁ - C₁₀ hydrocarbyl groups.

7. Conjugate according to claims 3 or 4, wherein a spiropyrane derivative is represented by formula **5:** 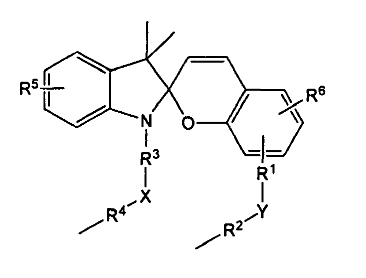 or 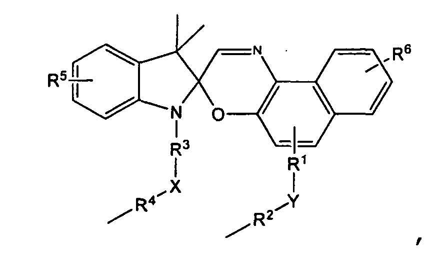 wherein
R¹, R², R³, R⁴ are independently selected from branched, un-branched or cyclic C₁-C₁₀ hydrocarbyl groups;
X, Y are independently selected from O, S, -NR⁷-, -C(O)NR⁷-, -C(S)NR⁷-, -NR⁷(C(O)-, and -NR⁷C(S)-;
R₇ is H, methyl, ethyl, or propyl;
R⁵ and R⁶ are independently selected from H, F, Cl, Br, I, branched, un-branched or cyclic C₁ - C₁₀ hydrocarbyl groups, NO₂, CF₃, OR⁹, OCOR⁹, COOR⁹, CH₂OR⁹, CHO, COR⁹, NR⁹R¹⁰ and SR⁹; R⁹ is H and branched, un-branched or cyclic C₁ - C₁₀ hydrocarbyl groups.

8. Conjugate according to claims 3 or 4, wherein a diarylethene derivative is represented by formula **6:** 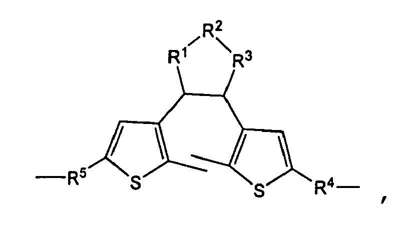 wherein
R¹, R², R³ are independently selected from CH₂, CHF, CF₂, O, S, and C(O);
R⁴ and R⁵ are independently selected from -R⁶-C(X)NR⁹-, -R⁶-R⁷-C(X)NR⁹-, -R⁶-R⁷-R⁸-C(X)NR⁹-, -R⁶-NR⁹-C(X)-, -R⁶-R⁷-NR⁹-C(X)-, -R⁶-R⁷-R⁸-NR⁹-C(X)-;
X is O or S;
R⁶, R⁷, and R⁸ are independently selected from branched, un-branched or cyclic C₁ - C₁₀ hydrocarbyl groups, -CH=C-, substitute or un-substituted phenyl or thiophene ring.
R⁹ is H, methyl, ethyl, and propyl.

9. Conjugate according to any of the preceding claims, wherein C is a cyclosporin A derivative or a FKBP binding moiety.

10. Conjugate according to claims 2 - 9, wherein the cyclosporin A derivative is represented by the formula **1:** 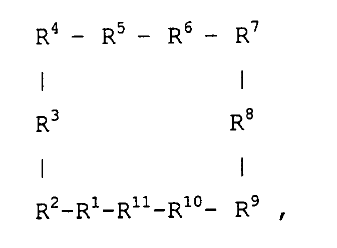 wherein R¹ is methyl(4R)-4-[(E)-2-Butenyl]-4-methyl-L-threonin (MeBMT) or dihydro-MeBMT;
R² is alpha-aminobutyric acid (Abu) or a halogenated derivative;
R³ is N-methyl glycine (Sar), or N-methyl-D-alanine, or a halogenated derivative;
R⁴, R⁶, R⁹, and R¹⁰ are N-methyl-L-leucin (MeLeu), or a halogenated derivative;
R⁵ is L-valine (Val) or a halogenated derivative;
R⁷ is L-alanine (Ala) or a halogenated derivative;
R¹¹ is N-methyl valine (MeVal) or a halogenated derivative; and wherein
R⁸ is a moiety represented by formula **2:** 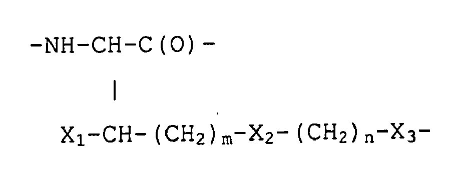 or 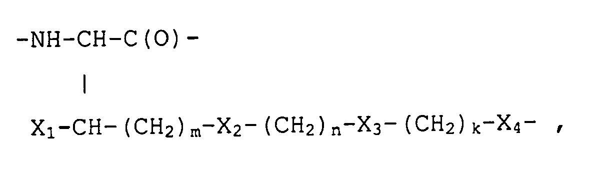
wherein
X₁ is H, methyl, and ethyl;
m is a value between 0 and 3;
X₂ is O or S;
n is a value between 0 and 4;
X₃ and X₄ is -NR₁₂-, -NR₁₂C(O)-, -NR₁₂C(S)-, -C(O)NR₁₂-, or -
C(S)NR₁₂-;
R₁₂ is H, methyl, ethyl, or propyl;
k is a value between 0 and 7.

11. Conjugate according to claim 10 , wherein
R¹ is methyl(4R)-4-[(E)-2-Butenyl]-4-methyl-L-threonin (MeBMT);
R² is alpha-aminobutyric acid (Abu);
R³ is N-methyl glycine (Sar);
R⁴, R⁶, R⁹, and R¹⁰ are MeLeu;
R⁵ is Val;
R⁷ is Ala; and
R¹¹ is MeVal.

12. Conjugate according to claims 2 - 9, wherein the cyclosporin A derivative is represented by formula **7:** 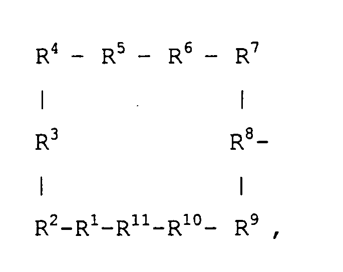 wherein
R¹ is MeBMT;
R² is Abu;
R³ is Sar;
R⁴, R⁶, R⁹, and R¹⁰ are MeLeu;
R⁵ is Val;
R⁷ is Ala;
R¹¹ is MeVal;
R⁸ is a moiety represented by formula **8:** 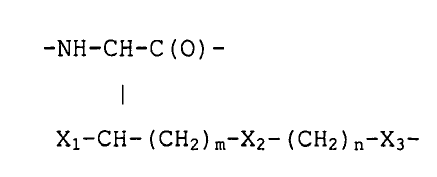
X₁ is H, methyl, and ethyl;
m is a value between 0 and 3;
X₂ is O or S;
n is a value between 0 and 4;
X₃ is -NR₁₂-, -NR₁₂C(O)-, -NR₁₂C(S)-, -C(O)NR₁₂-, or - C(S)NR₁₂-; and
R₁₂ is H, methyl, ethyl, or propyl.

13. Conjugate, according to claims 2 to 9, wherein the cyclosporin A derivative is represented by formula **12:** 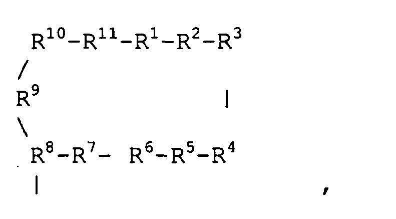 wherein R¹ is N-Methyl-(4R)-4-[(E)-2-butenyl]-4-methyl-L-threonin (MeBMT) or Dihydro-MeBMT;
R² is α-aminobutyric acid (Abu) or a fluorinated derivative thereof which is fluorinated on one or all positions of the side chain;
R³ is N-methyl-D-alanine (D-MeAla) or N-methyl-glycine (sarcosine (Sar)) or a fluorinated derivative thereof;
R⁴, R⁶, R⁹ and R¹⁰ are independently from one another N-methyl-L-leucine (MeLeu) or a fluorinated derivative thereof;
R⁵ is L-valine (Val) or a fluorinated derivative thereof;
R⁷ is L-alanine (Ala) or a fluorinated derivative thereof; R¹¹ is N-methyl-L-valin (MeVal) or a fluorinated derivative thereof; and
R⁸ is a moiety represented by formulae **13a and 13b**: 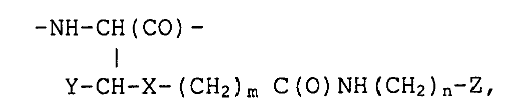
wherein X is O or S, Y is H or methyl, m has a value between 0 and 5,
n is an integer between 3 and 10,
Z is N-R¹²R¹³ or NHC(O)OR¹⁴, wherein
R¹² and R¹³ are independently from one another OH, a branched, un-branched or cyclic C₁ - C₁₀ hydrocarbyl group, preferably methyl, ethyl, n-propyl, iso-propyl, a substitute aromatic group or a photolysable group and
R¹⁴ is an acid/base labile or enzymatically cleavable group;
or 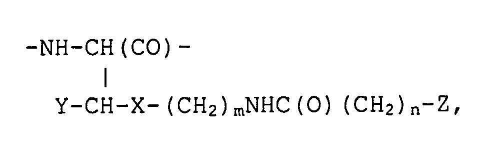 wherein X is O, S or CH₂,
Y is H or methyl,
m has a value between 0 and 5,
n is an integer between 3 and 10,
Z is N-R¹²R¹³ or NHC(O)OR¹⁴, wherein
R¹² is H, methyl, ethyl, n-propyl, iso-propyl or a photolysable group and
R¹⁹ is an acid/base labile or enzymatically cleavable group thereof.

14. Conjugate according to claims 4 - 13, wherein the FKBP binding moiety is represented by formula **9:** 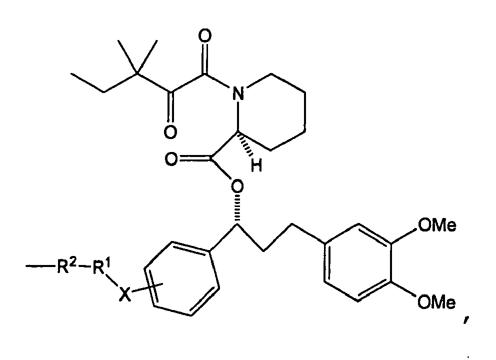 wherein
R¹ is a branched, un-branched or cyclic C₁ - C₁₀ hydrocarbyl groups;
R² is -C(X)-NR³- and -NR³-C(X)-;
R³ is H, methyl, ethyl, and propyl;
X is O or S.

15. Conjugate according to any of the preceding claims, wherein said conjugate is represented by formula **10:** 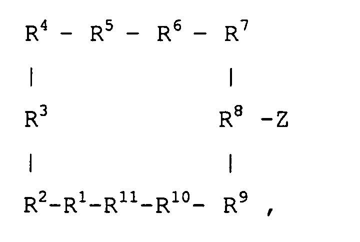 wherein
R¹ is MeBMT;
R² is Abu;
R³ is Sar;
R⁴, R⁶, R⁹, and R¹⁰ are MeLeu;
R⁵ is Val;
R⁷ is Ala;
R¹¹ is MeVal;
R⁸ is represented by formula **11:** 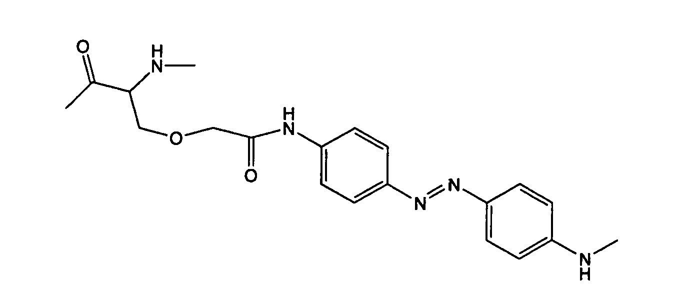
Z is a biotin, whose carboxylic acid group is coupled to the amino group on azobenzene to form an amide bond or Z is a FKBP binding ligand represented by formula **9:** 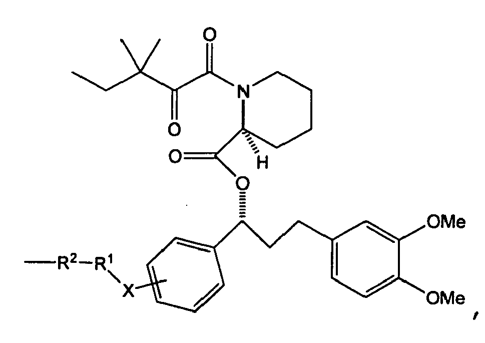 wherein
R¹ is a branched, un-branched or cyclic C₁ - C₁₀ hydrocarbyl groups;
R² is -C(X)-NR³- and -NR³-C(X)-;
R³ is H, methyl, ethyl, and propyl;
X is O or S.

16. Use of a conjugate according to claims 1 - 15 as a medicament.

17. Use of a conjugate according to claims 1 to 15 as a medicament for the treatment of a fungal infection.

18. Use of a conjugate according to claims 1 to 15 for the manufacture of a pharmaceutical composition for the treatment of a fungal infection.

19. Use of a conjugate according to claims 1 - 15, for the treatment of autoimmune and allergic diseases, inflammations, malign and benign tumor diseases, ocular diseases, fungal, viral, bacterial or other infections, cancer, vascular diseases, cardiovascular diseases, obesity, neurological disorders, degenerative neurological disorders, psychiatric diseases or conditions, depression, hormonal disorders, metabolic disorders, or diabetes, or as a drug for contraception.

20. Conjugate according to claims 1 to 15 for use in a method for treating a fungal infection.

21. Method for increasing the activity of a drug compound in cells, comprising the steps of:
a. transfecting cells with a conjugate according to claims 1-15,
b. irradiating the cells of step a) with light radiation.
